# EUROPÄISCHE PATENTSCHRIFT

(11) **EP 4 228 609 B1**
(45) Veröffentlichungstag und Bekanntmachung des Hinweises auf die Patenterteilung: **29.04.2026**
(21) Anmeldenummer: 22801036.9
(22) Anmeldetag: 04.10.2022
(51) Int. Cl.: A61K 9/50, A61K 33/06, A61K 9/00, A23L 33/16

(54) **DIREKTGRANULAT MIT KOMBINATION VON RETARDIERTEM UND SCHNELLFREISETZENDEM MAGNESIUM UND METHODEN ZUR HERSTELLUNG**
DIRECT GRANULES WITH A COMBINATION OF RETARDED AND FAST-RELEASING MAGNESIUM
GRANULÉS DIRECTS AVEC UNE COMBINAISON DE MAGNÉSIUM À LIBÉRATION RAPIDE ET RETARDÉE

(30) Priorität: 04.10.2021 EP 21200711
(43) Veröffentlichungstag der Anmeldung: 23.08.2023
(73) Patentinhaber: HERMES PHARMA GmbH, 82049 Pullach (DE)
(72) Erfinder: WEISS, Gerd, 81541 München (DE); HAALA, Josef, 82049 Pullach (DE); SCHIEMENZ, Wolfgang, 82049 Pullach (DE)
(74) Vertreter: Synergy IP Group AG
(86) Internationale Anmeldenummer: PCT/EP2022/077586
(87) Internationale Veröffentlichungsnummer: WO 2023/057451

(56) Entgegenhaltungen:
- KOEBERLE M ET AL: "Convenient magnesium supplementation: putting patients and consumers first", 27 August 2021 (2021-08-27), XP055898262, Retrieved from the Internet <URL:https://www.manufacturingchemist.com/news/article_page/Convenient_magnesium_supplementation_putting_patients_and_consumers_first/178345> [retrieved on 20220307]
- JANNIN VINCENT ET AL: "Hot-melt coating with lipid excipients", INTERNATIONAL JOURNAL OF PHARMACEUTICS, ELSEVIER, NL, vol. 457, no. 2, 23 October 2012 (2012-10-23), pages 480 - 487, XP028779490, ISSN: 0378-5173, DOI: 10.1016/J.IJPHARM.2012.10.026

## Beschreibung

### HINTERGRUND DER ERFINDUNG

Die vorliegende Erfindung betrifft neue Zusammensetzungen, insbesondere orale Direktgranulate, welche den Wirkstoff Magnesium enthalten, insbesondere eine Kombination aus einem Magnesium in retardierter Form sowie in einer schnellerfreisetzenden, nicht-retardierten Form. Die Zusammensetzungen werden unter anderem als Nahrungsergänzungsmittel und/oder als Arzneimittel in der Therapie oder Prävention von Magnesium-Mangelzuständen eingesetzt.

Um auch in Phasen erhöhten Bedarfs eine ausreichende Magnesium-Versorgung des Körpers zu sichern (bspw. bei Stress, Schwangerschaft, Mangelernährung, ausgeprägter sportlicher Betätigung, Diuretika-Therapie oder starkem Alkoholkonsum), empfiehlt es sich mitunter, dieses wichtige Mineral durch externe Quellen zu substituieren. Wie bei vielen wasserlöslichen Wirkstoffen besteht hierbei die Gefahr, dass der Körper überschüssiges Magnesium rasch mit dem Urin wieder ausscheidet, also jene Mengen, die er akut nicht verwenden oder in endogene Speicher einlagern kann. Zudem reagieren einige Personen mit Magenbeschwerden und/oder Durchfall auf höhere freie Magnesium-Konzentrationen im Magen-Darm-Trakt. Eine Formulierung mit zumindest in Teilen kontrollierter Freisetzung der enthaltenen Magnesiumgesamtdosis kann hierbei Abhilfe schaffen und bietet zudem weitere Vorteile wie u.a. eine geringere Einnahmefrequenz.

Für eine anhaltende, möglichst konstante Magnesium-Versorgung des Körpers wurden bereits Formulierungen mit kontrollierter Freisetzung entwickelt; beispielsweise retardierte Freisetzung (verzögerte Freisetzung) oder pulsatile Freisetzung (d.h. in mehreren "Pulsen" über einen längeren Zeitraum). So gibt es beispielsweise im Handel eine 3-lagige Magnesium-Mehrschichttablette mit B-Vitamin-Zusatz (Biolectra^{®} Magnesium 400 mg ultra 3-Phasen-Depot; Hermes Arzneimittel GmbH) welche als Nahrungsergänzungsmittel zur einmal täglichen Einnahme gedacht ist. Die drei Schichten enthalten (i) 200 mg Magnesium (+ Vit. B2 und B12) zur sofortigen Freisetzung, (ii) 90 mg Magnesium (+ Vit. B1) zur intermediären Freisetzung, sowie (iii) 110 mg Magnesium (+ Vit. B6) zur Langzeit-Freisetzung. Als Magnesiumverbin-dungen werden Magnesiumoxid, Magnesiumcarbonat sowie Magnesiumcitrat eingesetzt. Als Hilfsstoffe für die retardierte Freisetzung werden quellbare Polymere wie Hydroxypropylmethylcellulose (HPMC), Hydroxypropylcellulose (HPC) und Polyvinylpyrrolidon (PVP) eingesetzt. Leider sind diese Tabletten relativ groß aufgrund der hohen benötigten Mengen der Magnesium-Verbindungen sowie der Hilfsstoffe für deren retardierte Freisetzung (ca. 2 g Tablettengewicht), und dadurch für viele Anwender nicht problemlos oder nur unangenehm schluckbar.

Eine mögliche Abhilfe, bzw. Alternative, für große, schlecht schluckbare Tabletten bieten Brausetabletten oder Pulver zum Auflösen in Wasser oder auch sogenannte orale Direktgranulate. Granulate im engeren Sinne sind sogenannte Haufwerke, die mittels Aggregation oder Agglomeration von Pudern und/oder Pulvern hergestellt werden und somit meist grobkörniger als Puder und Pulver sind (wobei die Grenzen der Partikelgrößen insbesondere von Pulvern und Granulaten überlappen können). Gängige Verfahren zur Granulierung bzw. Aggregation sind dem Fachmann bekannt und in der Literatur beschrieben. Der Begriff "orale Direktgranulate" hingegen wird vom Fachmann breiter verstanden; es ist hierbei meist nicht von Belang, ob das Haufwerk tatsächlich durch eine Aggregation von Pulverpartikeln gewonnen wurde, oder ob einzelne oder mehrere Bestandteile des Direktgranulates anderweitig verarbeitet wurden. Es handelt sich bei oralen Direktgranulaten primär um oral verabreichbare, fließfähige Haufwerke, bevorzugt mit einer Teilchengröße im Bereich von 50 µm bis 500 µm, welche direkt, also in ihrer granulären bzw. partikulären, Form angewendet bzw. verabreicht werden; d.h. ohne hierfür Wasser oder andere trinkbare Flüssigkeiten zum Nachspülen zu benötigen. Sie können bspw. in den Mund und/oder auf die Zunge gestreut und dann leicht geschluckt werden.

Für solche oralen Direktgranulate ist allerdings das Mundgefühl von großer Bedeutung, welches unter anderem mitbestimmt wird durch strukturelle Eigenschaften (bspw. Texturen wie fettig, trocken, hart, formbar, klebrig, krümelig, etc.), durch geometrische Eigenschaften (groß/klein, rund/kantig, etc.) sowie durch Eigenschaften, welche mit dem im Mund-Rachenraum wahrgenommenen Wassergehalt (saftig, trocken, spröde, o.ä.), Temperaturempfindungen (wärmend, kühlend) oder der Reizung freier Nervenenden (scharf, brennend, prickelnd) zusammenhängen. Hier haben sich lipid-basierte Schmelzüberzüge bewährt, da sie häufig ein geringeres Fremdkörpergefühl auf der Zunge hinterlassen.

So beschreibt bspw. EP3042648B1 ein orales Direktgranulat mit Ascorbinsäure-Retardpartikeln aus kristalliner Ascorbinsäure und einem lipid-basierten Überzug, welcher in der Wirbelschicht als Schmelze auf die kristalline Ascorbinsäure aufgetragen wird. Darüber hinaus wird in der Publikation "Convenient magnesium supplementation: putting patients and consumers first" von Koeberle et al. (Manufacturing Chemist, 27.08.2021) auch ein schmelzüberzogenes, retardiertes Magnesium-Präparat angedacht; jedoch ohne konkrete Angaben die Herstellung und/oder verwendete Materialien betreffend.

Die Entwicklung eines industriell herstellbaren oralen Direktgranulats, welches eine kontrollierte Magnesium-Freisetzung mit sowohl retardierten als auch nicht-retardierten Anteilen ermöglicht, wurde bisher allerdings unter anderem dadurch erschwert, dass eine Mehrzahl der kommerziell erhältlichen Rohware von Magnesium-Formulierungen - anders als bspw. kristalline Ascorbinsäure - nicht hinreichend physikalisch stabil ist, um dem Schmelzüberzugsprozess (auch Hotmelt-Coating genannt) standzuhalten. Sehr häufig wurde hierbei beobachtet, dass zum Beispiel vorgranulierte Ware unter der mechanischen Belastung der Wirbelschicht und/oder dem prozess-inhärenten Wärmeinfluss zerfiel und dann die Filter des Wirbelschichtgeräts verstopfte, weit bevor der Schmelzüberzugsprozess ausreichend weit vorangeschritten war. Die Notwendigkeit sehr häufiger Filterwechsel, bzw. Filterreinigungsschritte machten diese Prozesse somit für den Industriemaßstab (bspw. ≥50 kg pro Charge) ungeeignet.

Zudem sind gerade solche Magnesium-Verbindungen, welche sich für den nicht-retardierten Dosisanteil aufgrund ihrer höheren Löslichkeit und Bioverfügbarkeit eher anbieten (z. Bsp. Magnesiumcitrate oder ähnliche Magnesiumsalze organischer Säuren), mehrheitlich sehr sauer oder geschmacklich wenig akzeptabel, und sind somit in einem oralen Direktgranulat schwieriger zu verabreichen, da dieses unverdünnt mit der Zunge in direkten Kontakt kommt.

Der vorliegenden Erfindung liegt somit die Aufgabe zugrunde, eine Zusammensetzung für Magnesiumverbindungen zur Verfügung zu stellen, welche industriell mit wenigen Schritten, wirtschaftlich hergestellt werden kann und eine kontrollierte Freisetzung von Magnesium mit sowohl retardierten als auch nicht-retardierten Anteilen ermöglicht. Die Zusammensetzung sollte leichter und angenehmer verabreichbar sein als gängige Tabletten oder Kapseln, bevorzugt auch ohne zusätzliche Flüssigkeitsgabe. Sie sollte zudem die Stabilität der Magnesiumverbindungen gewährleisten. Eine weitere Aufgabe der Erfindung besteht darin, Magnesium-haltige Kerne, welche für einen Lipidüberzug, und insbesondere für einen Schmelzüberzugsprozess mit Lipiden in der Wirbelschicht, vorgesehen sind - wie z. Bsp. kommerziell erhältliche Rohwaren Magnesium-haltiger Kerne - vorab so zu charakterisieren, dass deren Tauglichkeit, oder Eignung, für einen solchen Überzugsprozess bestimmt und exzessive Materialverluste durch instabiles Kernmaterial vermieden werden können.

Diese Aufgabe wird erreicht durch die erfindungsgemäße feste pharmazeutische oder nutrazeutische Zusammensetzung in Form eines oralen Direktgranulats wie nachfolgend beschrieben, sowie durch die Methoden und Verfahren zu deren Herstellung wie ebenfalls nachfolgend beschrieben.

### ZUSAMMENFASSUNG DER ERFINDUNG

In einem ersten Aspekt betrifft die vorliegende Erfindung eine feste pharmazeutische oder nutrazeutische Zusammensetzung gemäß Anspruch 1 in Form eines oralen Direktgranulats mit mindestens dualer Wirkstofffreisetzung, enthaltend folgende Komponenten:
(a) überzogene Retardpartikel mit einem Magnesium-haltigen Kern und einem Lipid-Überzug, wobei der Kern eine Magnesiumverbindung enthält oder aus dieser besteht, und wobei die Retardpartikel das enthaltene Magnesium in retardierter Weise freisetzen;
(b) nicht-retardierte Magnesium-haltige Partikel, wobei die Partikel eine Magnesium-verbindung enthalten oder aus dieser bestehen, und wobei die Partikel das enthaltene Magnesium in nicht-retardierter Weise freisetzen;
(c) einen oder mehrere wasserlösliche Hilfsstoffe ausgewählt aus der Gruppe der Zucker, Zuckeralkohole und Oligosaccharide; und
(d) optional einen oder mehrere weitere Hilfsstoffe;
dadurch gekennzeichnet, dass mindestens die Magnesium-haltigen Kerne der Retard-partikel der Komponente (a), bzw. eine Stichprobe hiervon, eine solche Abriebfestigkeit aufweisen, dass nach einer Vibrationssiebung über einen Zeitraum von 60 Minuten:
- der Feinanteil an Partikeln mit einem Siebdurchmesser von < 200 µm nach einer Siebzeit von 60 Minuten, t₆₀ₘᵢₙ, um maximal 100 Gew.-% höher ist gegenüber dem initialen Feinanteil dieses Siebdurchmessers nach einer Siebzeit von 5 Minuten, t₅ₘᵢₙ; und/oder
- der Anteil an Partikeln mit einem Siebdurchmesser von 200 µm ≤ x < 800 µm nach einer Siebzeit von 60 Minuten, t₆₀ₘᵢₙ, um maximal 18 Gew.-% niedriger ist gegenüber dem initialen Anteil dieses Siebdurchmessers nach einer Siebzeit von 5 Minuten, t₅ₘᵢₙ.

In einem zweiten Aspekt betrifft die vorliegende Erfindung eine Methode gemäß Anspruch 5 zur Charakterisierung eines Magnesium-haltigen Kerns für die Herstellung einer festen, lipid-überzogenen pharmazeutischen oder nutrazeutischen Zusammensetzung in der Wirbelschicht (optional die Herstellung der Retardpartikel der Komponente (a) der Zusammen-setzung gemäß dem ersten Aspekt der Erfindung), wobei der Kern eine Magnesium-verbindung enthält oder aus dieser besteht, und wobei die Methode dadurch gekennzeichnet ist, dass diese mittels einer Vibrationssiebung durchgeführt wird, und dadurch, dass Magnesium-haltige Kerne, welche für die Herstellung der festen, lipid-überzogenen pharmazeutischen oder nutrazeutischen Zusammensetzung in der Wirbelschicht als geeignet charakterisiert werden, bzw. eine Stichprobe hiervon, in diesem Test eine solche Abriebfestigkeit aufweist, dass nach der Vibrationssiebung über einen Zeitraum von 60 Minuten:
- der Feinanteil an Partikeln mit einem Siebdurchmesser von < 200 µm nach einer Siebzeit von 60 Minuten, t₆₀ₘᵢₙ, um maximal 100 Gew.-%, bevorzugt um maximal 50 Gew.-%, weiter bevorzugt um maximal 20 Gew.-%, höher ist gegenüber dem initialen Feinanteil dieses Siebdurchmessers nach einer Siebzeit von 5 Minuten, t₅ₘᵢₙ; und/oder
- der Anteil an Partikeln mit einem Siebdurchmesser von 200 µm ≤ x < 800 µm nach einer Siebzeit von 60 Minuten, t₆₀ₘᵢₙ, um maximal 18 Gew.-%, bevorzugt um maximal 15 Gew.-%, weiter bevorzugt um maximal 12 Gew.-%, niedriger ist gegenüber dem initialen Anteil dieses Siebdurchmessers nach einer Siebzeit von 5 Minuten, t₅ₘᵢₙ.

In einem dritten Aspekt betrifft die vorliegende Erfindung ein Verfahren gemäß Anspruch 11 zur Herstellung einer festen pharmazeutischen oder nutrazeutischen Zusammensetzung gemäß dem ersten Aspekt der Erfindung, wobei das Verfahren die folgenden Schritte enthält bzw. umfasst:
(i) Bereitstellen eines Magnesium-haltigen Kerns, wobei der Kern eine Magnesium- verbindung enthält oder aus dieser besteht;
(ii) Bereitstellen eines geschmolzenen Überzugsmaterials enthaltend ein Lipid;
(iii) Fluidisieren der Magnesium-haltigen Kerne;
(iv) Besprühen der fluidisierten Magnesium-haltigen Kerne mit dem geschmolzenen Überzugsmaterial;
(v) Abkühlen der überzogenen Magnesium-haltigen Kerne, so dass das Lipid erstarrt und Retardpartikel gemäß Komponente (a) erhalten werden, welche das enthaltene Magnesium in retardierter Weise freisetzen; und
(vi) Mischen der so als Komponente (a) erhaltenen Retardpartikel mit den folgenden weiteren Komponenten:
   (b) nicht-retardierte Partikel mit einem zweiten Magnesium-haltigen Kern, wobei der Partikel eine Magnesiumverbindung enthält oder aus dieser besteht, und wobei die Partikel das enthaltene Magnesium in nicht-retardierter Weise freisetzen;
   (c) einen oder mehrere wasserlösliche Hilfsstoffe ausgewählt aus der Gruppe der Zucker, Zuckeralkohole und Oligosaccharide; und
   (d) optional weiteren Hilfsstoffen;
dadurch gekennzeichnet, dass mindestens die Magnesium-haltigen Kerne der Retard-partikel der Komponente (a), bzw. eine Stichprobe hiervon, eine solche Abrieb-festigkeit aufweisen, dass nach einer Vibrationssiebung über einen Zeitraum von 60 Minuten:
- der Feinanteil an Partikeln mit einem Siebdurchmesser von < 200 µm nach einer Siebzeit von 60 Minuten, t₆₀ₘᵢₙ, um maximal 100 Gew.-% höher ist gegenüber dem initialen Feinanteil dieses Siebdurchmessers nach einer Siebzeit von 5 Minuten, t₅ₘᵢₙ; und/oder
- der Anteil an Partikeln mit einem Siebdurchmesser von 200 µm ≤ x < 800 µm nach einer Siebzeit von 60 Minuten, t₆₀ₘᵢₙ, um maximal 18 Gew.-% niedriger ist gegenüber dem initialen Anteil dieses Siebdurchmessers nach einer Siebzeit von 5 Minuten, t₅ₘᵢₙ.

In anderen Worten, in dem Herstellungsverfahren gemäß dem dritten Aspekt der Erfindung werden in Schritt (i) solche Magnesium-haltige Kerne bereitgestellt, welche mittels der Charakterisierungsmethode gemäß dem zweiten Aspekt der Erfindung als für die Herstellung einer festen, lipid-überzogenen pharmazeutischen oder nutrazeutischen Zusammensetzung in der Wirbelschicht geeignet charakterisiert wurden. Dies geschieht üblicherweise stichprobenartig, bspw. für ein bestimmte Charge der Magnesium-haltige Kerne.

In einem weiteren Aspekt der Erfindung betrifft die vorliegende Erfindung eine feste pharmazeutische oder nutrazeutische Zusammensetzung gemäß Anspruch 12 in Form eines oralen Direktgranulats mit mindestens dualer Wirkstofffreisetzung, welche(s) mittels des Verfahrens gemäß dem dritten Aspekt der Erfindung hergestellt wird.

### KURZBESCHREIBUNG DER ABBILDUNGEN

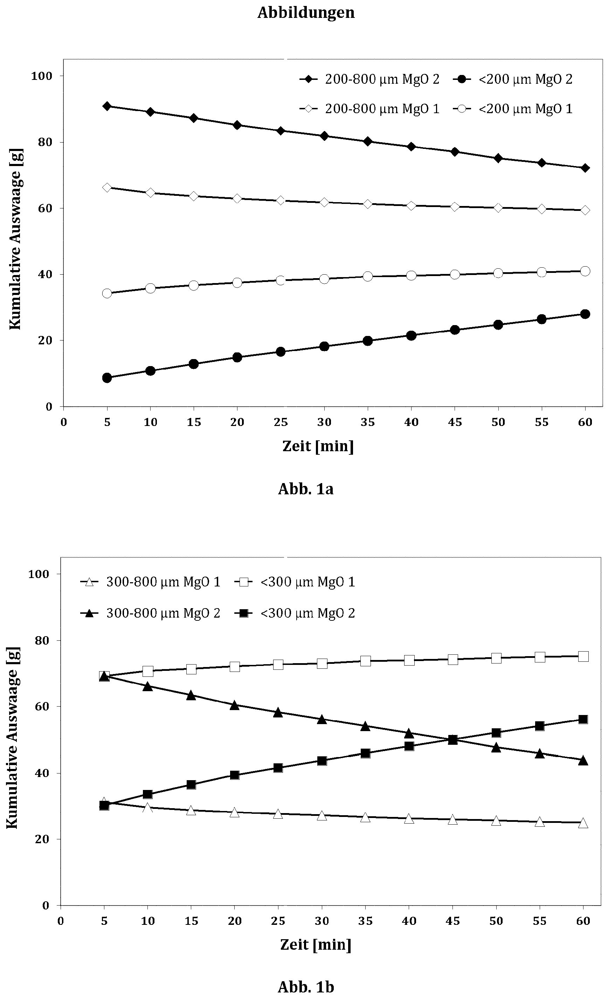

**Abb. 1a** und **Abb. 1b** zeigen die Ergebnisse der Vibrationssiebung über eine Zeit von 60 Minuten für zwei der getesteten Rohwaren von granuliertem Magnesiumoxid (MgO) Kernen (hier 'Heavy Magnesium Oxide EP' von Kyowa Chemical Industry Co., Ltd Japan, nachstehend als 'MgO 1' bezeichnet; sowie MagGran^{®} MO der Magnesia GmbH Deutschland; nachstehend ,MgO 2').

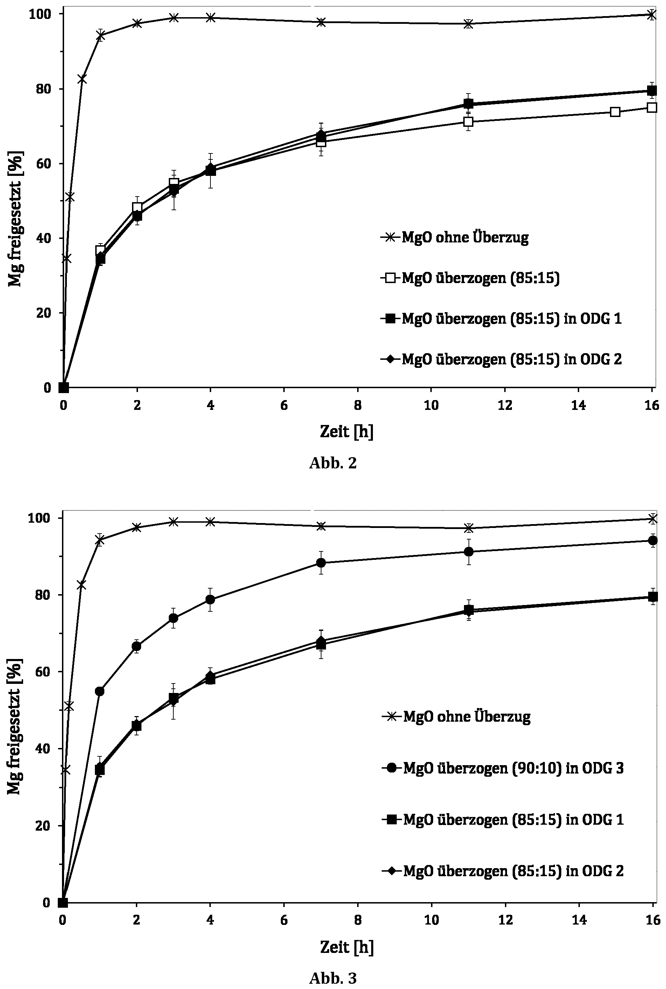

**Abb. 2** zeigt den Einfluss der Formulierung auf die Magnesium-Freisetzung in 0.1 N Salzsäure bei 37 °C für nicht überzogene MgO-Kerne, überzogene MgO-Kerne (Überzugslevel 15 Gew.-%) und erfindungsgemäße orale Direktgranulate (ODG), welche unter anderem diese überzogenen MgO-Kerne enthalten.
**Abb. 3** zeigt den Einfluss des Überzugslevels auf die Magnesium-Freisetzung in 0.1 N Salzsäure bei 37°C für nicht überzogene MgO-Kerne, und erfindungsgemäße orale Direktgranulate (ODG), welche unter anderem überzogene MgO-Kerne mit einem Überzugslevel von 10 Gew.-% (ODG 3) und 15 Gew.-% (ODG 1 und ODG 2) enthalten.

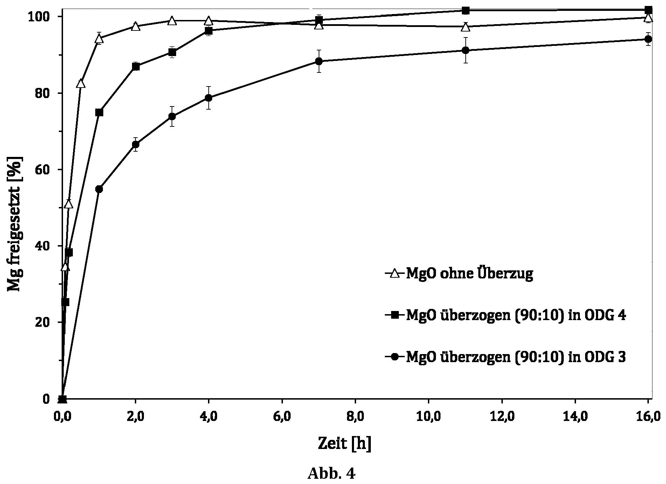

**Abb. 4** zeigt den Einfluss der Formulierung auf die Magnesium-Freisetzung in 0.1 N Salzsäure bei 37°C für nicht überzogene MgO-Kerne, und erfindungsgemäße orale Direktgranulate (ODG), welche unter anderem überzogene MgO-Kerne mit einem Überzugslevel von 10 Gew.-% enthalten sowie verschiedene Anteile nicht-retardiertes Magnesium, wobei ODG 3 weniger nicht-retardiertes Magnesium enthält als ODG 4.

### DETAILLIERTE BESCHREIBUNG DER ERFINDUNG

In einem ersten Aspekt betrifft die vorliegende Erfindung eine feste pharmazeutische oder nutrazeutische Zusammensetzung gemäß Anspruch 1 in Form eines oralen Direktgranulats mit mindestens dualer Wirkstofffreisetzung, enthaltend folgende Komponenten:
(a) überzogene Retardpartikel mit einem Magnesium-haltigen Kern und einem Lipid-Überzug, wobei der Kern eine Magnesiumverbindung enthält oder aus dieser besteht, und wobei die Retardpartikel das enthaltene Magnesium in retardierter Weise freisetzen;
(b) nicht-retardierte Magnesium-haltige Partikel, wobei die Partikel eine Magnesium-verbindung enthalten oder aus dieser bestehen, und wobei die Partikel das enthaltene Magnesium in nicht-retardierter Weise freisetzen;
(c) einen oder mehrere wasserlösliche Hilfsstoffe ausgewählt aus der Gruppe der Zucker, Zuckeralkohole und Oligosaccharide; und
(d) optional einen oder mehrere weitere Hilfsstoffe;
dadurch gekennzeichnet, dass mindestens die Magnesium-haltigen Kerne der Retard-partikel der Komponente (a), bzw. eine Stichprobe hiervon, eine solche Abriebfestigkeit aufweisen, dass nach einer Vibrationssiebung über einen Zeitraum von 60 Minuten:
- der Feinanteil an Partikeln mit einem Siebdurchmesser von < 200 µm nach einer Siebzeit von 60 Minuten, t₆₀ₘᵢₙ, um maximal 100 Gew.-% höher ist gegenüber dem initialen Feinanteil dieses Siebdurchmessers nach einer Siebzeit von 5 Minuten, t₅ₘᵢₙ; und/oder
- der Anteil an Partikeln mit einem Siebdurchmesser von 200 µm ≤ x < 800 µm nach einer Siebzeit von 60 Minuten, t₆₀ₘᵢₙ, um maximal 18 Gew.-% niedriger ist gegenüber dem initialen Anteil dieses Siebdurchmessers nach einer Siebzeit von 5 Minuten, t₅ₘᵢₙ.

Im Zusammenhang mit der Erfindung bezeichnet der Begriff 'Retardpartikel' solche Partikel, die einen Wirkstoff, hier eine Magnesium-Verbindung, und mindestens einen Hilfsstoff in Form eines Überzugs enthalten, und die so aufgebaut sind, dass der Hilfsstoffüberzug eine langsame Freisetzung des Wirkstoffs bewirkt (sog. Reservoir-Systeme). Im Falle der vorliegenden Erfindung wird diese Retardierung durch einen Lipid-Überzug gewährleistet.

Gemäß Anspruch 1, ist der Lipid-Überzug der Retardpartikel der Komponente (a) durch ein Schmelzbeschichtungsverfahren auf die Magnesium-haltigen Kerne aufgebracht, oder in anderen Worten, es handelt sich um einen Lipid-Schmelzüberzug.

Da die vorliegende Erfindung feste pharmazeutische oder nutrazeutische Zusammensetzungen in Form von oralen Direktgranulat betrifft, meint der Begriff 'Kern' bzw. ,Magnesium-haltiger Kern' wie hierin verwendet Kernmaterialien in Haufwerk-Form, bspw. granuläre Rohwaren in trockener, fließfähiger Partikelform, mit mittleren Korngrößen von üblicherweise ca. 600 µm oder kleiner.

Des Weiteren bezeichnet im Zusammenhang mit der hierin beschriebenen Erfindung der Begriff ,Abriebfestigkeit' nicht nur solche Feinanteile eines Haufwerks, welche ausschließlich durch Oberflächenabrieb entstanden sind, sondern auch solche, die durch Zerfall bzw. Bruch der zugrundeliegenden, getesteten Partikel bzw. Kerne entstanden sind. Dies liegt vor allem darin begründet, dass diese beiden Anteile (Abrieb im engeren Sinne und Bruch) im Anschluss an die Siebanalyse nicht unterscheidbar sind. Daher sind die Begriffe Abriebfestigkeit und Bruchfestigkeit für diese Erfindung als synonym zu verstehen.

In einer spezifischen Ausführungsform weisen mindestens die Magnesium-haltigen Kerne der Retardpartikel der Komponente (a), bzw. eine Stichprobe hiervon, eine solche Abriebfestigkeit auf, dass nach einer Vibrationssiebung über einen Zeitraum von 60 Minuten der Feinanteil an Partikeln mit einem Siebdurchmesser von < 200 µm nach einer Siebzeit von 60 Minuten, t₆₀ₘᵢₙ, um maximal 50 Gew.-%, bevorzugt um maximal 20 Gew.-%, höher ist gegenüber dem initialen Feinanteil dieses Siebdurchmessers nach einer Siebzeit von 5 Minuten, t₅ₘᵢₙ; und/oder der Anteil an Partikeln mit einem Siebdurchmesser von 200 µm ≤ x < 800 µm nach einer Siebzeit von 60 Minuten, t₆₀ₘᵢₙ, um maximal 15 Gew.-%, bevorzugt um maximal 12 Gew.-%, niedriger ist gegenüber dem initialen Anteil dieses Siebdurchmessers nach einer Siebzeit von 5 Minuten, t₅ₘᵢₙ.

In einer weiteren Ausführungsform weisen mindestens die Magnesium-haltigen Kerne der Retardpartikel der Komponente (a), bzw. eine Stichprobe hiervon, eine solche Abriebfestigkeit auf, dass nach einer Vibrationssiebung über einen Zeitraum von 60 Minuten der Anteil an Partikeln mit einem Siebdurchmesser von < 300 µm nach einer Siebzeit von 60 Minuten, t₆₀ₘᵢₙ, um maximal 55 Gew.-%, bevorzugt um maximal 35 Gew.-%, weiter bevorzugt um maximal 15 Gew.-%, höher ist gegenüber dem initialen Anteil dieses Siebdurchmessers nach einer Siebzeit von 5 Minuten, t₅ₘᵢₙ; und/oder der Anteil an Partikeln mit einem Siebdurchmesser von 300 µm ≤ x < 800 µm nach einer Siebzeit von 60 Minuten, t₆₀ₘᵢₙ, um maximal 30 Gew.-%, bevorzugt um maximal 27 Gew.-%, weiter bevorzugt um maximal 24 Gew.-%, niedriger ist gegenüber dem initialen Anteil dieses Siebdurchmessers nach einer Siebzeit von 5 Minuten, t₅ₘᵢₙ.

Natürlich können optional auch die nicht-retardierten Magnesium-haltigen Partikel, bzw. eine Stichprobe hiervon, eine solche Abriebfestigkeit wie oben beschrieben aufweisen. Dies wäre unter anderem von Vorteil, wenn bspw. das Auftragen eines wasserlöslichen, nicht-retardierenden Überzugs auf die nicht-retardierten Magnesium-haltigen Partikel oder ein Granulieren derselben in der Wirbelschicht angedacht ist.

Genauere Parameter der Vibrationssiebung, mit welcher die oben beschriebene Abriebfestigkeit bestimmt wird, bzw. die Magnesium-haltigen Kern charakterisiert werden, sind weiter unten für den zweiten Aspekt der Erfindung noch in detaillierterer Form beschrieben; all diese Parameter sind explizit auf die Zusammensetzung gemäß dem ersten Aspekt der Erfindung anwendbar.

In einer beispielhaft zusammenfassenden Ausführungsform kann die Vibrationssiebung bspw. mit 100 g der Magnesium-haltigen Kerne (gesamte zu siebende Menge, welche auf das oberste Sieb des Turmes, bspw. ein 800 µm Sieb, aufgegeben wird) und je 1 g Siebhilfe pro Siebboden, sowie bei einer Vibrationsamplitude von 80/min, durchgeführt werden. Hierbei kann ein handelsüblicher Siebturm mit Siebböden von ca. 20 cm Durchmesser zum Einsatz kommen, bspw. der AS 200 basic der Firma Retsch GmbH. Als Siebhilfe können hierbei bspw. abriebfeste Silicagel-Kugeln verwendet werden, welche einen Durchmesser von ca. 2-6 mm, oder ca. 2-5 mm, oder ca. 2-4 mm aufweisen (bspw. ca. 3 mm), und eine Schüttdichte von ca. 650-850 kg/m³, oder ca. 700-800 kg/m³ (bspw. eine Schüttdichte von ungefähr 750 kg/m³). Die Dauer der erfindungsgemäßen Vibrationssiebung wurde auf 60 min festgelegt (mit mindestens einer Zwischenwägung bei 5 min), da dies ungefähr dem Zeitrahmen eines gängigen Schmelzbeschichtungsverfahrens entspricht (eine Variante des Aufbringens von Lipidüberzügen); sprich so lange sind die Kerne im Allgemeinen dem mechanischen Stress der Wirbelschicht, sowie den üblicherweise erhöhten Temperaturen eines Schmelzbeschichtungsverfahrens, ausgesetzt und müssen eine hinreichende Abriebfestigkeit aufweisen. In Fällen, die ein deutlich längeres Sprühverfahren erfordern, könnte theoretisch eine längere Siebdauer als 60 Minuten angedacht werden. Allerdings ist dies häufig nicht nötig, da die Magnesium-haltigen Kerne im Allgemeinen nach 60 Minuten bereits so weit überzogen sind, dass der Überzug die Kerne stabilisiert und insbesondere die Auswirkungen des mechanischen Stresses auf das Kernmaterial immer geringer werden.

Wie oben an erwähnt war in der Vergangenheit die Entwicklung eines industriell herstellbaren oralen Direktgranulats mit schmelzüberzogenen Magnesium-Kernen dadurch erschwert bzw. unmöglich gemacht worden, dass viele, der kommerziell erhältlichen Magnesium-Formulierungen nicht hinreichend stabil schienen, um dem Schmelzüberzugsprozess standzuhalten. So zerfiel bspw. vorgranulierte Ware und/oder wies starken Abrieb auf, und verstopfte somit die Filter des Wirbelschichtgeräts, weit bevor der Schmelzüberzugsprozess ausreichend weit vorangeschritten war, was sehr häufige Filterwechsel, bzw. Filterreinigungsschritte, nötig machte, und somit den Prozess ungeeignet für den Industriemaßstab (bspw. ≥50 kg pro Charge). Zudem waren die entstehenden Auflagerungen von verkrustetem Kern-Abrieb und Sprühmaterial (bspw. Triglyceride wie Glyceroltripalmitat und/oder Glyceroltristearat) auf den Innenwänden des Wirbelschicht-Geräts so ausgeprägt und fest, dass stets sehr zeitaufwändige Reinigungsschritte nötig waren.

Im Rahmen extensiver Versuche zur Herstellung des oralen Direktgranulates gemäß dem ersten Aspekt der Erfindung hatten die Erfinder zunächst kommerziell erhältliche Magnesium-haltige Kerne gefunden, welche diese Probleme nicht aufwiesen, bzw. zu einem deutlich geringeren Grad, und die somit für ein Schmelzbeschichtungsverfahren mit Lipiden (bspw. mit Triglyceriden wie Glyceroltripalmitat und/oder Glycerol-tristearat) geeignet waren. Allerdings fanden sich auch hier Unterschiede, nicht nur zwischen den Rohwaren verschiedener Hersteller, sondern auch zwischen verschiedenen Chargen desselben Herstellers, so dass erneut unklar blieb, ob eine bestimmte Rohware der Magnesium-haltigen Kerne für ein Schmelzbeschichtungsverfahren, insbesondere für ein Schmelzbeschichtungsverfahren im Industriemaßstab, geeignet ist oder nicht.

Mit standardisierten Abriebtestern wie bspw. dem Friabilator ließen sich die Magnesium- haltigen Kerne der vorliegenden Erfindung nicht bzw. nicht hinreichend akkurat unterscheiden in solche Kerne, die einem Lipid-Überzugsverfahren, insbesondere einem Schmelz-Überzugsverfahren mit Lipiden, in der Wirbelschicht standhalten konnten (auch im Industriemaßstab), und solche, die nicht standhielten und sich für das Überzugsverfahren durch ihren hohen Abrieb als ungeeignet erwiesen.

Erst der Einsatz der Vibrationssiebung, und insbesondere der Einsatz der Vibrationssiebung mit der beschriebenen Siebhilfe, ermöglichte es, die mechanischen Belastungen eines Wirbelschicht-Coating-Prozesses zu simulieren, insbesondere die eines Wirbelschicht-Coating-Prozesses bei dem ein Schmelzüberzug (Hotmelt-Coating) aufgebracht wird. Auf diese Weise ist es möglich, vorab die Abriebfestigkeit der für die Herstellung der Retardpartikel vorgesehenen Magnesium-haltigen Kerne zu bestimmen und somit eine Aussage zu treffen, ob diese eine ausreichende Stabilität für den Überzugsprozess aufweisen. Dadurch können unnötige Materialverluste vermieden werden sowie die Leerzeiten (in denen bspw. das Überzugsequipment gereinigt werden muss) reduziert werden, da nur solche Chargen der Magnesium-haltigen Kerne überzogen werden, die die oben beschriebenen Anforderungen an die Abriebfestigkeit aufweisen, oder in anderen Worten, nur solche Magnesium-haltigen Kerne (oder Chargen hiervon) überzogen werden, die in der Methode gemäß dem zweiten Aspekt der Erfindung als geeignet erscheinen. Für die Erfindung ist es hierbei unerheblich, ob die Abriebfestigkeit der für die Herstellung der Retardpartikel vorgesehenen Magnesium-haltigen Kerne (ausreichend oder nicht ausreichend) das Resultat von Chargen-Variabilitäten eines einzelnen Anbieters ist, oder durch Chargen verschiedener Anbieter verursacht ist.

In einer Ausführungsform werden die erfindungsgemäßen Magnesium-haltigen Kerne der Retardpartikel mittels Trockenkompaktierung hergestellt; manchmal auch als Walzenkompaktierung bezeichnet.

In einer der bevorzugten Ausführungsform weisen die Magnesium-haltigen Kerne der Retardpartikel eine mediane Teilchengröße (D50), gemessen mittels dynamischer Bildanalyse zwischen 150 µm und 300 µm auf, bevorzugt zwischen 165 µm und 285 µm, weiter bevorzugt zwischen 180 µm und 270 µm.

Alle hierin verwendeten Partikelgrößen-Angaben - sowohl gemessene Werte als auch aus gemessenen Werten berechnete/abgeleitete Werte - beziehen sich auf Werte, die mittels dynamischer Bildanalyse ermittelt wurden; z. Bsp. mit dem Camsizer^{®} XT Gerät der Retsch Technology GmbH, Haan, Deutschland ausgestattet mit der X-Jet Plug-in Kartusche sowie der dazugehörigen Analyse-Software. Der Camsizer^{®}-Aufbau verwendet eine dynamische Bildgebungstechnik, bei der Partikelproben durch Druckluft zerstreut, durch einen von gepulsten LED-Lichtquellen beleuchteten Spalt geleitet und ihre Bilder (genauer ihre Projektionen) hierbei von zwei Digital-Kameras aufgezeichnet und dann in Bezug auf Größe und Form analysiert werden. Auf diese Weise können eine Vielzahl von Längen- und Breitendeskriptoren für die Partikel bestimmt werden, einschließlich der mittleren Partikelgröße, der medianen Partikelgröße (D50), sowie die sog. D10- und D90-Werte. Das Gerät Camsizer^{®} XT wird für die Partikelgrößenbestimmung gemäß der Erfindung bevorzugt, da es die genaue und reproduzierbare Analyse von Partikelgrößen von feinen Pulvern bis hinunter zu 1 µm ermöglicht. Dies sollte jedoch nicht so missverstanden werden, dass die Verwendung von Laserbeugung oder anderen etablierten Methoden zur Partikelgrößenbestimmung im erforderlichen Mikrometerbereich ausgeschlossen wäre; jedoch sind in Fällen, in denen die mit anderen Verfahren ermittelten Partikelgrößen von den mittels Camsizer^{®} ermittelten Werten abweichen, die mit dem Camsizer^{®}- ermittelten Werte maßgebend.

Die oben genannten Partikelgrößen der Magnesium-haltigen Kerne sind insofern vorteilhaft, dass die daraus resultierenden entsprechenden Retardpartikel auch nach Auftrag des zur Retardierung benötigten Überzugs noch eine angenehme Partikelgröße behalten, die bei einer direkten oralen Applikation nicht als grobkörnig oder anderweitig unangenehm empfunden wird. Die Partikelgröße von oralen Direktgranulaten sollte im Allgemeinen 600 µm, bevorzugt 500 µm; nicht überschreiten, um ein Fremdkörpergefühl im Mund zu vermeiden und den Kaureflex gering zu halten.

Auch Rohwaren Magnesium-haltiger Kerne, die mittels Trockenkompaktierung hergestellt sind, bieten hierfür Vorteile, da sie oftmals keine, oder höchstens geringfügige, Beimengungen von Hilfsstoffen enthalten, und die Magnesium-haltigen Kerne somit in konzentrierter, Magnesium-reicher Form vorliegen, und so ebenfalls kleine Partikelgrößen bieten. Allerdings haben die Erfinder auch mit diesen Rohwaren die oben beschriebene Beobachtung gemacht, dass die Abriebfestigkeit der trockengranulierten Rohwaren oft nicht ausreichend war, um Schmelzbeschichtungsverfahren, auch solche im Industrie-Maßstab, zu ermöglichen. Die Erfinder können hierzu lediglich spekulieren, ob möglicherweise Unterschiede in der Restfeuchte des verpressten Materials, unterschiedliche Pressdrücke, die Art der Zerkleinerung der Press-Schülpe oder andere Faktoren zu Unterschieden in der Abriebfestigkeit der Rohware führen und so deren Tauglichkeit für Schmelzbeschichtungsverfahren (mit)beeinflussen.

In einer weiteren bevorzugten Ausführungsform weisen die Magnesium-haltigen Kerne der Retardpartikel eine unimodale Partikelgrößenverteilung auf; sprich eine Partikelgrößenverteilung, die im Verteilungsdiagramm nur einen einzelnen Peak aufweist. Dies deutet im Allgemeinen auf eine überwiegend einheitliche Partikelgröße hin. Weiterhin bevorzugt sind Ausführungsformen, in denen die Magnesium-haltigen Kerne der Retardpartikel eine so enge Partikelgrößenverteilung aufweisen, dass der Quotient (D90-D10) / D50 unter 1,60 liegt, bevorzugt unter 1,50, und weiterhin bevorzugt unter 1,40. Da oberhalb von D90 und unterhalb von D10 eher die Ausreißer der Partikelgrößenverteilung zu finden sind, deutet ein Quotient(D90-D10) / D50, der nahe an eins liegt, darauf hin, dass die Mehrzahl der Partikel eine Größe nahe der medianen Partikelgröße (D50) aufweist.

Es ist weiterhin vorteilhaft, wenn mindestens die Magnesiumverbindung in den Retardpartikeln der Komponente (a) in ihrer wasserfreien Form einen Magnesiumgehalt von 15 Gew.-% oder mehr aufweist, bevorzugt 25 Gew.-% oder mehr, weiterhin bevorzugt 50 Gew.-% oder mehr. Dies ist insofern von Vorteil, dass auf diese Weise die Menge und/oder Größe der "Retard-Kerne" geringgehalten werden kann, und die hieraus hergestellten Retardpartikel auch nach Auftrag Überzugs noch eine angenehme Partikelgröße behalten wie oben beschrieben. Optional kann natürlich auch die Magnesiumverbindung in den nicht-retardierten Partikeln der Komponente (b) in ihrer wasserfreien Form einen Magnesiumgehalt von 15 Gew.-% oder mehr, bevorzugt 25 Gew.-% oder mehr, weiterhin bevorzugt 50 Gew.-% oder mehr aufweisen. In einer Ausführungsform ist die Magnesiumverbindung in den Retardpartikeln ausgewählt aus Magnesiumoxid (MgO), Magnesiumcarbonat (MgCO₃), Magnesiumchlorid (MgCl₂), Magnesiumhydrogenphosphat (MgHPO₄) und/oder Magnesiumacetat (Mg(CH₃COO)₂).

Optional kann der Kern der Retardpartikel einen oder mehrere Hilfsstoffe enthalten, bspw. im Falle einer vorgranulierten Magnesiumhaltigen Rohware eine kleine Menge eines wasserlöslichen Bindemittels, das von Herstellerseite der Rohware zugesetzt sein kann. Wie oben erwähnt ist hierbei jedoch vorteilhaft, wenn möglichst wenig Hilfsstoffe zugesetzt sind, um auf diese Weise kleine Partikelgrößen bei hohem Magnesiumgehalt zu gewährleisten.

In einer Ausführungsform ist die Magnesiumverbindung im Kern der Retardpartikel Magnesiumoxid (MgO). In einer spezifischen Ausführungsform besteht der Kern in den Retardpartikeln im Wesentlichen aus Magnesiumoxid (MgO).

In einer der bevorzugten Ausführungsform enthält der Überzug der Retardpartikel ein Lipid mit einem Schmelzpunkt von mindestens 50 °C, oder besteht im Wesentlichen hieraus; bevorzugt ein Lipid mit einem Schmelzpunkt von mindestens 60 °C. Im Falle, dass der Überzug mehrere Lipide enthält, hat mindestens eines der Lipide einen Schmelzpunkt von mindestens 50 °C, bevorzugt mindestens 60 °C. Ferner, im nicht unüblichen Fall, dass ein Lipid keinen scharfen Schmelzunkt, sondern einen Schmelzbereich aufweist, ist unter dem Schmelzpunkt im Sinne der Erfindung die Untergrenze des Schmelzbereichs zu verstehen, also die Temperatur, bei der das Lipid während des Aufheizens zu schmelzen beginnt.

Die Erfinder haben festgestellt, dass sich insbesondere Lipide mit einem höheren Schmelzpunkt von mindestens 50 °C, bevorzugt mindestens 60 °C, hervorragend für orale Direktgranulate eignen. Diese Lipide sind zum einen ausreichend fest, um den Magnesium-haltigen Kern der Retardpartikel stabil zu umhüllen und werden bei Körpertemperatur noch nicht (an)geschmolzen. Im Unterschied zu Lipiden mit Schmelzpunkten unter 50 °C oder ähnlichen hydrophoben Umhüllungen aus Wachsen oder Silikonen hinterlassen die erfindungsgemäßen Lipide zudem keinen seifigen oder fettigen (Geschmacks)Eindruck im Mund, was für eine Zusammensetzung in Form eines oralen Direktgranulats wichtig ist, da dieses üblicherweise länger im Mund verbleibt als bspw. eine perorale Tablette und dort nicht durch zusätzliche Flüssigkeiten verdünnt wird.

Gleichzeitig werden die erfindungsgemäßen Lipidüberzüge im Vergleich mit konventionellen Polymer-Überzügen (wie bspw. aus Celluloseethern) im Mund und/oder auf und unter der Zunge subjektiv als weniger hart und weniger "plastikartig" wahrgenommen; erzeugen also weniger Fremdkörpergefühl. All dies trägt maßgeblich zu dem gewünschten angenehmen Mundgefühl des erfinderischen oralen Direktgranulates bei.

Der Lipidüberzug kann optional einen oder mehrere weitere Hilfsstoffe enthalten, mit denen seine Eigenschaften hinsichtlich Verarbeitbarkeit, Geschmack, Aussehen, Stabilität oder Wirkstofffreisetzung angepasst werden. Dies können z. Bsp. wasserlösliche Substanzen sein, Substanzen mit einer geringeren Schmelztemperatur, quellbare Substanzen und/oder Netzmittel wie bspw. Polysorbate (z. Bsp. Tween^{®}). Ein Ziel dieser Hilfsstoffe im Lipidüberzug - sofern eingesetzt - ist es, die Stabilität des Filmes zu unterstützen und ggf. variablere Freisetzungsprofile zu ermöglichen. Beispiele für Hilfsstoffe, welche die Verarbeitbarkeit von Lipiden und Lipidmischungen potenziell verbessern können, sind Emulgatoren, vor allem solche mit mittlerem oder niedrigem HLB-Wert (hydrophilic-lipophilic balance) von etwa 12 oder weniger. Prinzipiell sollten hierbei natürlich nur solche Substanzen gewählt werden, die zum einen den Geschmackseindruck nicht negativ beeinflussen, und zum anderen kein Inkompatibilitätsrisiko mit weiteren Überzugsbestandteilen, im Speziellen mit dem/den Lipid(en), aufweisen (bspw. zu Phasentrennungen führen könnten).

Bevorzugt besteht der Überzug jedoch überwiegend aus einem oder mehreren Lipiden, das heißt zu mindestens 70 Gew.-%. Ebenso bevorzugt sind Überzüge mit mindestens 80 Gew.-% Lipid(en), mit mindestens 90 Gew.-% Lipid(en), oder solche, die praktisch ausschließlich aus Lipid(en) bestehen.

In einer Ausführungsform handelt es sich bei den Lipiden im Überzug der Retardpartikel um Triglyceride (auch Triacylglyceride, Glycerol-Triester oder seltener auch Neutralfette genannt), sprich dreifache Ester von Glycerol mit drei Fettsäuremolekülen.

In einer weiteren Ausführungsform enthält der Überzug der Retardpartikel mindestens 70 Gew.-%, oder mindestens 80 Gew.-%, oder mindestens 90 Gew.-% eines Triglycerids. In einer spezifischen Ausführungsform handelt es sich um Triglyceride natürlicher Fettsäuren, bspw. Palmitinsäure, Stearinsäure oder Arachidon-säure. In einer weiteren spezifischen Ausführungsform handelt es sich bei den Lipiden im Überzug um möglichst reine Formen einfacher Triglyceride, sprich Triglyceride die mindestens 80 Gew.-% und bevorzugt mindestens 90 Gew.-% eines einfachen Triglycerids enthalten, und nur wenig gemischte- und/oder Partialglyceride. Beispiele für solche einfachen Triglyceride sind Glyceroltripalmitat, Glyceroltristearat oder Glyceroltriarachidat. Optional können die Triglyceride auch miteinander gemischt werden.

In einer der bevorzugten Ausführungsformen enthält der Überzug der Retardpartikel Glyceroltripalmitat und/oder Glyceroltristearat, oder besteht im Wesentlichen hieraus; z. Bsp. mit einem Überzug aus Dynasan^{®}116 oder 118. In einer spezifischen Ausführungsform enthält der Überzug der Retardpartikel min. 70 Gew.-%, oder min. 80 Gew.-%, oder min. 90 Gew.-% Glyceroltripalmitat und/oder Glyceroltristearat. In einer der bevorzugten Ausführungsformen, besteht der Überzug der Retardpartikel im Wesentlichen aus Glyceroltripalmitat und/oder Glyceroltristearat. Einer der Vorzüge von Überzügen, welche zu mindestens 90 Gew.-% aus Glyceroltripalmitat und/oder Glyceroltristearat wie bspw. Dynasan^{®}116 oder 118 bestehen, ist, dass sie synthetisch gewonnen werden und häufig reiner sind als die Mehrzahl natürlicher oder partial-synthetischer Lipide; also z. Bsp. weniger gemischte Triglyceride oder Partialglyceride aufweisen, und/oder klarer definierte Schmelzpunkte haben. Dies wirkt sich vorteilhaft auf ihre Verarbeitung aus, da bspw. eine zu sprühende Schmelze nicht unnötig hoch erhitzt werden muss, um alle Bestandteile des Lipids vollständig aufzuschmelzen.

Im Gegensatz hierzu muss bei Lipiden mit breiteren Schmelzbereichen üblicherweise noch oberhalb des oberen Wertes dieses Schmelzbereichs gearbeitet werden, um eine homogene Zusammensetzung der Schmelze zu gewährleisten und ein mögliches Verstopfen der Sprühdüse(n) zu verhindern. Solche 'übererhitzten' Schmelzen härten auf den zu besprühenden Magnesium-haltigen Kernen dann langsamer aus, formen also den gewünschten Überzug weniger zügig. Zudem kann die Agglomerationstendenz durch das langsamere Aushärten erhöht sein. Weiterhin sind Entmischungen der Schmelze während des Erstarrens auf den zu besprühenden Magnesium-haltigen Kernen möglich, wenn die höherschmelzenden Komponenten der Schmelze zuerst aushärten.

Darüber hinaus liegen die Überzüge mit mindestens 90 Gew.-% Glyceroltripalmitat und/oder Glyceroltristearat nach der Verarbeitung bereits in ihrer stabileren β-Modifikation vor, so dass sich die Freisetzung während der Lagerung üblicherweise nicht, bzw. nur marginal ändert. Die so erhaltenen Retardpartikel sind also deutlich lagerstabiler.

In einer weiteren bevorzugten Ausführungsform besteht der Überzug der Retardpartikel im Wesentlichen aus Glyceroltristearat. Insbesondere Glyceroltristearat (Schmelzpunkt Mₚ ca. 70-73 °C) hat sich in Versuchen aufgrund seines höheren Schmelzpunktes als besonders angenehm im Mund herausgestellt und hinterlässt überraschenderweise deutlich weniger unangenehm seifige und/oder fettige (Geschmacks) Eindrücke als andere Triglyceride mit einem Schmelzpunkt deutlich oberhalb der Körpertemperatur wie z. Bsp. Trilaurin (Mₚ ca. 44-47 °C). Zudem ist Glyceroltristearat deutlich geschmacksneutraler als eine Vielzahl natürlicher oder partial-synthetischer Lipide. Es hinterlässt bspw. anders als gehärteter Rindertalg (Mₚ ca. 60 °C) oder gehärtetes Rizinusöl (Mₚ 79-80 °C) keinen ,deftigen' oder respektive 'kratzenden' Geschmackseindruck auf der Zunge. Auch viele neutrale gehärtete Öle (wie Raps oder Soja) können unter Umständen in Direktgranulaten als geschmacklich unangenehm bzw. unpassend wahrgenommen werden und harmonieren nicht mit frischen und/oder fruchtigen Aromen wie Orange, Zitrone, Kirsche, Beere, Tropenfrüchte oder dergleichen.

Die Freisetzung lässt sich neben der Zusammensetzung des Überzugs, auch über dessen Stärke (oder Dicke) steuern. Die Stärke ergibt sich wiederum aus der Auftragsmenge des Überzugsmaterials und aus der Oberfläche der zu überziehenden Partikel. Je nach der Größe der Magnesium-haltigen Kerne und der sich daraus ergebenden Oberfläche ist demnach die Auftragsmenge des Überzugsmaterials zu wählen, mit dem die gewünschte Retardierung erzielt wird. In einer der bevorzugten Ausführungsformen liegt das Gewichtsverhältnis zwischen Kern und Überzug in den Retardpartikeln zwischen 60:40 und 95:5, oder zwischen 70:30 und 90:10; bspw. 75:25, 80:20, 85:15 oder 90:10. Das konkrete Gewichtsverhältnis zwischen Kern und Überzug kann nach Maßgabe der angestrebten Freisetzungskinetik bzw. Retardierung und der Zusammensetzung angepasst werden. Hierbei ist zu beachten, dass sich die benötigte Menge an Überzugsmaterial selbst für ein und denselben Lipidüberzug leicht ändern kann, wenn bspw. die Partikelform und -größe und/oder die Porosität des Überzugsgutes variieren.

In einer der bevorzugten Ausführungsformen sind die Retardpartikel mit einem Überzug versehen, der zu mindestens 90 Gew.-% aus Glyceroltripalmitat und/oder Glycerol-tristearat besteht, und bei dem das Gewichtsverhältnis zwischen Kern und Überzug zwischen 60:40 und 95:5 liegt. In einer ebenfalls bevorzugten Ausführungsform sind die Retardpartikel mit einem Überzug versehen, der zu mindestens 90 Gew.-% aus Glyceroltristearat besteht, oder der optional im Wesentlichen hieraus besteht, und bei dem das Gewichtsverhältnis zwischen Kern und Überzug zwischen 70:30 und 90:10 liegt, bevorzugt zwischen 80:20 und 90:10.

Im Zusammenhang mit der oben erwähnten zu überziehenden Oberfläche ist zudem zu beachten, dass Magnesium-haltige Kerne mit unzureichender Abriebfestigkeit, wie bspw. von den Erfindern in früheren Schmelzbeschichtungsversuchen verwendet, nicht allein aufgrund der sehr arbeits- und zeitintensiven Arbeitsschritte kritisch sind (verstopfte Filtern, häufige und schwierige Reinigung), sondern vor allem ein Problem darstellen, da sich durch die fortschreitende Zerkleinerung eines unzureichend stabilen Kernmaterials in der Wirbelschicht die zu überziehende Oberfläche in kaum kalkulierbarer Weise verändert und somit ein reproduzierbares Schmelzbeschichtungsverfahren nahezu unmöglich wird.

*In vitro* lässt sich das Freisetzungsprofil gemäß den gängigen Arzneibüchern mit einer genormten Freisetzungsapparatur unter standardisierten Bedingungen bestimmen. Erfindungsgemäß wird das Freisetzungsprofil bevorzugt in einer Freisetzungs-Apparatur mit Blattrührer (USP Dissolution Apparatus 2) bspw. in 900 mL 0.1 N Salzsäure (HCl) bei 37 °C und einer Rührgeschwindigkeit von 75 UPM bestimmt. Unter diesen Bedingungen zeigt die erfindungsgemäße Zusammensetzung eine Retardierung über mindestens ca. 4-6 Stunden, vorzugsweise über mindestens ca. 6-8 Stunden; d.h. vor Ablauf des jeweiligen Zeitraums ist der Wirkstoff noch nicht vollständig freigesetzt.

In einer der bevorzugtem Ausführungsformen ist die Stärke des Überzugs in den Retardpartikeln so gewählt, dass die Retardpartikel innerhalb von 4 Stunden, bevorzugt innerhalb von 5 Stunden, weiter bevorzugt innerhalb von 6 Stunden, nicht mehr als 85 % des in ihnen enthaltenen Magnesiums freisetzen, gemessen in einer Freisetzungs-Apparatur mit Blattrührer (USP Dissolution Apparatus 2) in 900 mL 0.1 N Salzsäure bei 37 °C und einer Rührgeschwindigkeit von 75 UPM. Längere Retardierungen durch die Retard-partikel sind häufig nicht mehr zielführend, da sich die Partikel nach 8-10 h normaler-weise im Dickdarm befinden, wo sowohl die Resorptionsoberfläche als auch das für die Freisetzung benötigte Wasser deutlich reduziert sind.

Ebenso bevorzugt sind Ausführungen, welche nach 4 Stunden zwischen 50 % und 80 % des Wirkstoffs freigesetzt haben, und/oder nach 6 Stunden zwischen 60 % und 90 %, und/oder nach 8 Stunden zwischen 65 % und 100 %.

Um die gewünschte Retardierung über ca. 6-8 h und/oder von nicht mehr als 85 % innerhalb von 4 Stunden mit den Retardpartikeln zu erzielen, sollte das Gewichtsverhältnis zwischen Kern und Lipidüberzug zwischen 60:40 und 90:10 liegen. Vorteilhaft ist auch ein Gewichtsverhältnis zwischen 80:20 und 95:5, z. Bsp. 85:15 oder 90:10. Hierbei sind Gewichtsverhältnisse oberhalb von 80:20, wie bspw. 85:15, 90:10 oder 95:5, auch deshalb vorteilhaft, weil geringere Auftragsmengen des Überzugsmaterials typischerweise mit kürzeren Prozesszeiten einhergehen.

Während der Überzug der Retardpartikel vorrangig die gewünschte retardierte Freisetzung des Wirkstoffs sicherstellt, fungiert dieser aber auch als Geschmacksmaskierung für die in den Kernen enthaltene Magnesium-verbindung, da diese aufgrund der Retardierung kaum oder gar nicht auf der Zunge freigesetzt wird. Gerade für einige der sehr sauer oder geschmacklich wenig akzeptabel schmeckenden Magnesiumverbindungen, wie bspw. einigen Magnesium-Citraten oder Magnesium-Acetat, kann dies unter Umständen vorteilhaft sein.

Erfindungsgemäß ist die Zusammensetzung in Form eines oralen Direktgranulats so aufgebaut, dass sie neben den überzogenen Magnesium-Retardpartikeln der Komponente (a) auch einen Anteil nicht-retardierter Magnesium-haltige Partikel als Komponente (b) enthalten. Diese sind vorrangig als schnell-freisetzende Initialdosis gedacht. Üblicherweise ist die Menge bzw. Dosis an nicht-retardiertem Magnesium kleiner als die der überzogenen Magnesium-Retardpartikeln; bspw. etwa 5-100 mg nicht-retardiertes Magnesium in Kombination mit Dosis 50-700 mg Magnesium-Retardpartikeln, z. Bsp. ca. 8 mg Initialdosis plus 500 mg retardiertes Magnesium, oder ca. 50 mg Initialdosis plus 350 mg retardiertes Magnesium. Die beiden Teildosen sollten hierbei so gewählt sein, dass sie in der Summe eine Tagesdosis von ca. 800 mg Magnesium, bevorzugt ca. 600 mg, nach Möglichkeit nicht überschreiten.

In einer Ausführungsform ist die Magnesiumverbindung in den nicht-retardierten Partikeln der Komponente (b) ausgewählt aus Magnesiumoxid (MgO), Magnesiumcarbonat (MgCO₃), Magnesiumacetat (Mg(CH₃COO)₂), Magnesiumhydrogenphosphat (MgHPO₄) Magnesiumhydrogencitrat (C₆H₆MgO₇), Trimagnesiumdicitrat (C₁₂H₁₀Mg₃O₁₄), Magnesiumpidolat (C₁₀H₁₂MgN₂O₆), Magnesium N-Acetyltaurinat (C₈H₁₆MgN₂O₈S₂) und/oder Magnesium-bis(hydrogen-L-glutamat), sowie deren Hydraten. In einer spezifischen Ausführungsform ist die Magnesiumverbindung in den nicht-retardierten Partikeln der Komponente (b) ausgewählt aus Magnesiumhydrogencitrat und/oder Trimagnesiumdicitrat. In einer spezifischeren Ausführungsform bestehen die nicht-retardierten Partikel der Komponente (b) im Wesentlichen aus Magnesium-hydrogencitrat und/oder Trimagnesiumdicitrat.

In einer Ausführungsform enthalten die Retardpartikel der Komponente (a) eine andere Magnesiumverbindung als die nicht-retardierten Partikel der Komponente (b). So kann bspw. in einer der bevorzugten Ausführungsformen der Kern der Retardpartikel Magnesiumoxid enthalten, und die nicht-retardierten Partikel der Komponente (b) enthalten Magnesiumhydrogencitrat und/oder Trimagnesiumdicitrat. Weiterhin bevorzugt sind Ausführungsformen, in denen der Kern der Retardpartikel im Wesentlichen aus Magnesiumoxid besteht, und die nicht-retardierten Partikel der Komponente (b) im Wesentlichen aus Magnesiumhydrogencitrat und/oder Trimagnesiumdicitrat bestehen.

Trimagnesiumdicitrat ist ein nicht-saures Salz der Zitronensäure, und kann somit geschmacklich neutraler sein als bspw. Magnesiumhydrogencitrat, was unter Umständen für ein orales Direktgranulat vorteilhaft sein kann.

In einer Ausführungsform weisen die Retardpartikel der Komponente (a), und optional die nicht-retardierten Partikel der Komponente (b), eine mediane Teilchengröße (D50), gemessen mittels dynamischer Bildanalyse (z. Bsp. mittels Camsizer^{®} XT), von ≤ 500 µm auf, bevorzugt ≤ 450 µm, weiter bevorzugt ≤ 400 µm. Wie oben erwähnt, sollte die Partikelgröße von oralen Direktgranulaten im Allgemeinen 600 µm, bevorzugt 500 µm, nicht überschreiten, um ein Fremdkörpergefühl im Mund zu vermeiden und den Kaureflex gering zu halten. Mit anderen Worten, die Mehrzahl der Partikel (mindestens 80 %, bevorzugt mindestens 90 %.) sollte ein Sieb mit einer Maschenweite von ca. 600 µm passieren. In einer bevorzugten Ausführungsform der Erfindung haben daher die Retardpartikel der Komponente (a) - und optional die nicht-retardierten Partikel der Komponente (b) - eine mediane Teilchengröße (D50), gemessen mittels dynamischer Bildanalyse, zwischen 100 µm und 500 µm, bevorzugt zwischen 150 µm und 450 µm, weiterhin bevorzugt zwischen 200 µm und 400 µm.

In einer Ausführungsform machen die Retardpartikel der Komponente (a) zwischen 15 und 85 Gew.-%, oder zwischen 25 und 75 Gew.-%, oder zwischen 30 und 70 Gew.-%, oder zwischen 30 und 60 Gew.-% der festen pharmazeutischen oder nutrazeutischen Zusammensetzung gemäß dem ersten Aspekt aus; bspw. 30-35 Gew.-% oder 55-60 Gew.-%. Die Wahl ist hierbei vorrangig davon geleitet, welcher Anteil der Magnesium-Gesamtdosis in der Zusammensetzung retardiert freigegeben werden soll und wie hoch die Einmaldosis für die Applikation gewählt wird.

Neben den Retardpartikeln der Komponente (a) und den nicht-retardierten Magnesium-haltigen Partikeln der Komponente (b) enthält die Zusammensetzung in Form eines oralen Direktgranulats mindestens einen wasserlöslichen Hilfsstoff gemäß Kompo-nente (c), mit dem die anderen Komponenten vermischt werden. Es handelt sich hierbei erfindungsgemäß um Hilfsstoffe, die gemäß Definition des Europäischen Arzneibuchs löslich, leicht löslich oder sehr leicht löslich sind in Wasser (d.h. 1 g zu lösender Substanz benötigt zur Auflösung nicht mehr als 30 mL Wasser, respektive nicht mehr als 10 mL oder weniger als 1 mL Wasser) und die aus der Gruppe der Zucker, Zuckeralko-hole und Oligosaccharide ausgewählt sind. Bei der Verabreichung des oralen Direkt-granulates fungieren die wasserlösliche Hilfsstoffe der Komponente (c) quasi als äußere Phase einer schluckbaren, in-situ gebildeten Suspension, mit deren Hilfe die Retard-partikel leichter geschluckt werden können. Bevorzugt werden daher schmackhafte, wasserlösliche Hilfsstoffe wie Zucker, Zuckeralkohole oder wasserlösliche Vertreter der Oligosaccharide gewählt. Dies unterstützt auch die Compliance bei einer dauerhaften Anwendung des Direktgranulates. Zwar wäre es theoretisch möglich, die Retardpartikel der Kompo-nente (a) und die Magnesiumhaltigen Partikel der Komponente (b) auch allein, ohne weitere Hilfsstoffe zu verabreichen, allerdings würden diese sich in dem Fall nicht ganz so leicht und angenehm schlucken lassen, länger im Mund verbleiben und vom Anwender möglicherweise zerkaut werden, wodurch der Retard-Effekt aufgehoben würde.

Die Menge an Hilfsstoffen, insbesondere die Menge der Komponenten (c) und (d), sollte hierbei vorzugsweise so gewählt sein, dass die Gesamtmenge an oralem Direktgranulat pro Dosis nicht mehr als ca. 3 g beträgt, oder nicht mehr als etwa 2,5 g oder 2,0 g, da größere Pulver- bzw. Granulat-Mengen im Mund und auf der Zunge nur noch schwierig gleichmäßig zur durchfeuchten sind. Sofern nötig kann eine Einzeldosis in zwei oder mehreren Teilen (z. Bsp. 2-3 Sachets mit nicht mehr als ca. 3 g, bevorzugt nicht mehr als ca. 2 g, Direktgranulat) eingenommen werden.

In einer Ausführungsform enthält die feste pharmazeutische oder nutrazeutische Zusammensetzung gemäß dem ersten Aspekt mindestens 20 Gew.-%, bevorzugt mindestens 23 Gew.-%, weiterhin bevorzugt mindestens 25 Gew.-% der Hilfsstoffe gemäß Komponente (c).

In einer Ausführungsform enthalten die Hilfsstoffe der Komponente (c) in der festen pharmazeutischen oder nutrazeutischen Zusammensetzung gemäß dem ersten Aspekt mindestens 60 Gew.-%, bevorzugt mindestens 75 Gew.-%, weiterhin bevorzugt mindestens 85 Gew.-%, eines in Wasser löslichen Zuckers oder Zuckeralkohols; optional bestehen sie im Wesentlichen hieraus. Hierbei kann der in Wasser lösliche Zucker oder Zuckeralkohol ausgewählt sein aus Saccharose, Sorbitol, Xylitol, Erythritol, Maltitol, Isomaltitol, Mannitol, und Mischungen derselben. In einer spezifischen Ausführungsform sind die oralen Direktgranulate so zusammengesetzt sind, dass die Hilfsstoffe der Komponenten (c) und (d) mindestens 50 Gew.-%, oder mindestens 55 Gew.-% Sorbitol oder Xylitol enthalten, oder im Falle einer Mischung aus Sorbitol und Xylitol mindestens 50 Gew.-%, oder mindestens 55 Gew.-%, dieser Mischung. In einzelnen Ausführungs-formen können die oralen Direktgranulate auch so zusammengesetzt sein, dass die Hilfsstoffe der Komponenten (c) und (d) sogar mindestens 85 Gew.-%, oder mindestens 90 Gew.-% Sorbitol oder Xylitol enthalten, oder im Falle einer Mischung aus Sorbitol und Xylitol mindestens 85 Gew.-%, oder mindestens 90 Gew.-%, dieser Mischung.

Durch diese Gewichtsanteile der löslichen Zucker, Zuckeralkohole oder Oligosaccharide wird ein ausreichendes Volumen an flüssigem wohlschmeckendem Suspensionsmedium für die erfindungsgemäße Zusammensetzung im Mund des Anwenders gebildet und die Schluckbarkeit sowie die Compliance des Anwenders positiv beeinflusst.

Optional können der Zusammensetzung in Form eines oralen Direktgranulats neben den Zuckern, Zuckeralkoholen und/oder Oligosacchariden noch weitere Hilfsstoffe gemäß der Komponente (d) zugesetzt sein, die beispielsweise die Viskosität der sich in-situ bildenden Granulat-Suspension auf der Zunge leicht erhöhen, die den Speichelfluss anregen, oder die den Geschmack beeinflussen oder die Riesel- bzw. Fließfähigkeit des Granulates regulieren. Optional können auch die Hilfsstoffe gemäß (d) wasserlöslich sein (also löslich, leicht löslich oder sehr leicht löslich gemäß der Definition des Europäischen Arzneibuchs; d.h. 1 g zu lösender Substanz benötigt zur Auflösung nicht mehr als 30 mL Wasser, respektive nicht mehr als 10 mL oder weniger als 1 mL Wasser).

In einer Ausführungsform enthält die feste pharmazeutische oder nutrazeutische Zusammensetzung gemäß dem ersten Aspekt beispielsweise einen sauren Hilfsstoff als Bestandteil der Hilfsstoffe gemäß Komponente (d). Bevorzugte saure Hilfsstoffe sind Zitronensäure, Weinsäure, Äpfelsäure, Bernsteinsäure, sowie saure Salze derselben, etwa Mononatriumcitrat. Die Erfinder haben festgestellt, dass der Einsatz dieser sauren Substanzen - vor allem in Kombination mit den löslichen Zuckern und Zuckeralkoholen in den bevorzugten Mengen - eine zusätzliche Anregung des Speichelflusses hervorruft, durch den die Zusammensetzung, und insbesondere die typischerweise etwas größeren Retardpartikel darin, optimal dispergiert werden und besonders leicht zu schlucken sind. In einer spezifischen Ausführungsform enthält die Zusammensetzung als weitere Hilfsstoffe gemäß Komponente (d) Zitronensäure, Mononatriumcitrat und/oder Dinatriumcitrat, insbesondere Zitronensäure in Kombination mit Mononatriumcitrat.

In einer weiteren Ausführungsform kann die Zusammensetzung in Form oraler Direktgranulate so zusammengesetzt sein, dass die Hilfsstoffe der Komponente (d) viskositätserhöhende Substanzen als schluckbarkeitsfördernde Hilfsstoffe enthalten, z. Bsp. Celluloseether wie Carmellose-Natrium (auch Natrium-Carboxymethylcellulose genannt; typischerweise in der nicht quervernetzten Form) oder modifizierte Stärken wie bspw. Lycatab^{®} PGS (eine vollständig vorverkleisterte Maisstärke). Ihre Konzentration sollte so gewählt werden, dass sie einerseits das Mundgefühl des Direktgranulates verbessern, und die in situ daraus entstehende Retardpartikel-Suspension stabilisieren, andererseits aber das Schlucken nicht erschweren; sprich die auf der Zunge geformte Suspension sollte sämig sein, nicht schleimig oder klebrig.

Weiter optional können die oralen Direktgranulate so zusammengesetzt sein, dass die Hilfsstoffe der Komponente (d) Gleitmittel enthalten; z. Bsp. dem Fachmann bekannte Fließregulierungs- und Schmiermittel wie hochdisperses Siliciumdioxid, Magnesiumstearat und/oder Stearinsäure. Diese Substanzen dienen dazu, das orale Direktgranulat streufähig bzw. fließ- oder rieselfähig zu machen, so dass es leicht abgefüllt und auch leicht wieder aus Verpackungen entnommen werden kann. Magnesiumstearat wird hierbei bevorzugt, wird aber explizit nicht mit zu den Magnesium-haltigen Komponen-ten (a) oder (b) gezählt, da Magnesiumstearat, sofern verwendet, nur in vergleichsweise geringen Mengen zum Einsatz kommt (≤0,50 Gew.-%, bevorzugt ≤0,30 Gew.-%, weiter bevorzugt ≤0,25 Gew.-%), um so einen wachsartigen Eindruck im Mund zu vermeiden.

Des Weiteren können Aromen, Süßstoffe, Geschmackskorrigenzien, Farbstoffe usw. als Bestandteil der Hilfsstoffe gemäß Komponente (d) verwendet werden, um die organoleptischen Eigenschaften der erfindungsgemäßen Zusammensetzung anzupassen. So können die oralen Direktgranulate bspw. so zusammengesetzt sein, dass die Hilfsstoffe neben den löslichen Zuckern, Zuckeralkoholen oder Oligosacchariden der Komponente (c) weitere Süßungsmittel bzw. Süßstoffe (bspw. Aspartam, Acesulfam-K, Sucralose oder dgl.), und/oder Aromen (Zitrone, Orange, Tropenfrüchte, etc.) enthalten. Gängige und verträgliche Süßungsmittel und Aromen sind dem Fachmann hinreichend bekannt, z. Bsp. aus oralen pädiatrischen Formulierungen.

Optional können die oralen Direktgranulate zusätzlich zu den Komponenten (a) bis (d), und insbesondere zusätzlich zum Magnesium, noch weitere nutrazeutische Substanzen enthalten, wie z. Bsp. Mineralien und/oder Vitamine. Optional können auch diese in retardierter Form vorliegen, bspw. mit retardierenden Überzügen und/oder eingebettet in eine retardierende Matrix. In einer Ausführungsform enthält die Zusammensetzung bspw. Vitamine der B-Reihe und/oder Vitamin D. In einer spezifi-schen Ausführungsform enthält die Zusammensetzung bspw. Magnesium in Kombination mit Vitamin D3 und B12. In einer weiteren spezifischen Ausführungsform enthält die Zusammensetzung bspw. Magnesium in Kombination mit Vitamin B1, B2, B6 und B12.

In einer Ausführungsform sind die Mengen der Retardpartikel der Komponente (a) und der nicht-retardierten Partikel der Komponente (b) so gewählt, dass innerhalb von 4 Stunden, bevorzugt innerhalb von 5 Stunden, weiter bevorzugt innerhalb von 6 Stunden, nicht mehr als 85 % des in der Zusammensetzung enthaltenen Magnesiums freisetzen, gemessen in einer Freisetzungs-Apparatur mit Blattrührer (USP Dissolution Apparatus 2) in 900 mL 0.1 N Salzsäure bei 37 °C und einer Rührgeschwindigkeit von 75 UPM. Um dies zu gewährleisten sind typischerweise mindestens 60 Gew.-%, oder mindestens 75 Gew.-%, oder mindestens 85 Gew.-%, des gesamten in der Zusammen-setzung enthaltenen Magnesiums in den Retardpartikeln der Komponente (a) enthalten. Alternativ oder zusätzlich hierzu enthält die Zusammensetzung typischerweise mindestens 5 Gew.-%, oder mindestens 10 Gew.-%, oder mindestens 12 Gew.-%, und bis maximal 25 Gew.-%, des gesamten in der Zusammensetzung enthaltenen Magnesiums in nicht-retardierter Form.

Optional können einzelne oder alle der Substanzen oder Hilfsstoffe gemäß Komponen-ten (b), (c) und/oder (d), welche den Retardpartikeln der Komponente (a) beigemischt werden, in granulierter Form vorliegen. Dies kann z. Bsp. in Fällen vorteilhaft sein, um einen potenziell störenden Feinstaubanteil zu reduzieren und/oder wenn die Mischung aus den Retardpartikeln der Komponente (a) und die Komponenten gemäß (b), (c) und/oder (d) zur Entmischung neigen (z. Bsp., wenn die Partikelgröße(n) aller oder einzelner Stoffe in den Komponenten (b), (c) und/oder (d) deutlich unter der Partikelgröße der Retardpartikel liegen). Eine Angleichung oder Annäherung der Partikelgrößen der Komponenten (b), (c) und/oder (d) an die Partikelgröße der Retardpartikel der Komponente (a) durch Granulierung kann diesen Entmischungstendenzen entgegenwirken und so ein gleichmäßiges Ausgießen des oralen Direktgranulats gewährleisten. In einer Ausführungsform weisen auch die beigemengten Hilfsstoffpartikel der Komponenten (b), (c) und/oder (d), eine mediane Teilchengröße (D50), gemessen mittels dynamischer Bildanalyse, zwischen 100 µm und 500 µm auf, bevorzugt zwischen 150 µm und 450 µm, weiterhin bevorzugt zwischen 200 µm und 400 µm.

In einer der bevorzugten Ausführungsformen ist das orale Direktgranulat zudem so zusammengesetzt, dass die Hilfsstoffe der Komponenten (c) und (d) nicht mehr als 5 Gew.-% von in Wasser schwerlöslichen Hilfsstoffen enthalten; d.h. umgekehrt sollten rund 95 Gew.-% oder mehr der Hilfsstoffe löslich sein. Dies ist wünschenswert, um das Fremdkörpergefühl im Mund so gering wie möglich zu halten.

In einer der bevorzugten Ausführungsformen sind Einzeldosen der Zusammensetzung in Stickpacks, Sachets, Glasampullen oder Plastikampullen verpackt, um dem Anwender so die Dosierung des Magnesiums zu erleichtern. Stickpacks sind hierbei besonders bevorzugt, da sie leicht und bruchsicher transportiert werden können und auch beim Öffnen durch Aufreißen üblicherweise eine kleinere Schüttöffnung bieten - und somit leichter vollständig in den Mund geleert werden können - als die vergleichbar aufgebauten, aber meist breiter dimensionierten Sachets. Zudem bieten Stickpacks aufgrund ihrer Laminierung eine einfache Möglichkeit zum Schutz des Direktgranulates vor Licht und Sauerstoff und stabilisieren so das in der Zusammensetzung enthaltene Magnesium zusätzlich.

In einer Ausführungsform enthalten die Einzeldosen der Zusammensetzung typischerweise zwischen 50 und 700 mg Magnesium, bevorzugt zwischen 100 und 650 mg, weiter bevorzugt zwischen 150 und 600 mg oder zwischen 350 und 550 mg, z. Bsp. ca. 400 mg, ca. 500 mg oder ca. 510 mg. In einer spezifischen Ausführungsform enthält eine Einzeldosis der Zusammensetzung ca. 350 mg Magnesium in den Retardpartikeln der Komponente (a) und ca. 50 mg Magnesium in den nicht-retardierten Partikeln der Komponente (b); bspw. ca. 350 mg Magnesium in Form von Magnesium-oxid in den Retardpartikeln und ca. 50 mg Magnesium in Form von nicht-retardiertem Magnesiumhydrogencitrat, oder alternativ ca. 350 mg Magnesium in Form von Magnesiumoxid gemischt mit ca. 42 mg Magnesium in Form von nicht-retardiertem Magnesiumhydrogencitrat und ca. 8 mg Magnesium in Form von nicht-retardiertem Trimagnesiumdicitrat. In einer weiteren spezifischen Ausführungsform enthält eine Einzeldosis der Zusammensetzung ca. 510 mg Magnesium in den Retardpartikeln der Komponente (a) und ca. 8 mg Magnesium in den nicht-retardierten Partikeln der Komponente (b); bspw. ca. 500 mg Magnesium in Form von Magnesiumoxid und ca. 10 mg Magnesium in Form von nicht-retardiertem Trimagnesiumdicitrat.

In einer der bevorzugten Ausführungsformen wird eine Zusammensetzung in Form eines oralen Direktgranulats bereitgestellt, enthaltend:
Retardpartikel mit einem Magnesium-haltigen Kern und einem Überzug welcher zu mindestens 90 Gew.-% aus Glyceroltripalmitat und/oder Glyceroltristearat besteht, bspw. Dynasan^{®}116 oder 118, wobei das Gewichtsverhältnis zwischen Kern und Überzug zwischen 70:30 und 90:10 liegt;
nicht-retardierte Magnesium-Partikel;
mindestens einen oder mehrere wasserlösliche Hilfsstoffe ausgewählt aus Saccharose, Sorbitol, Xylitol, Erythritol, Maltitol, Isomaltitol, Mannitol und Mischungen derselben; mindestens einen oder mehrere speichelanregende Hilfsstoffe, ausgewählt aus Zitronensäure, Mononatriumcitrat, Dinatriumcitrat und Mischungen derselben;
optional einen oder mehrere schluckbarkeitsfördernde Hilfsstoffe ausgewählt aus Celluloseethern oder modifizierten Stärken und Mischungen derselben;
weiterhin optional ein Gleitmittel ausgewählt aus Magnesiumstearat, Stearinsäure und hochdispersem Siliciumdioxid oder Mischungen derselben; mindestens ein Aroma; sowie optional weitere Süßstoffe oder Süßungsmittel, wobei der Gewichtsanteil der Retardpartikel im oralen Direktgranulat zwischen 25 % und 75 % liegt.

In einer weiteren bevorzugten Ausführungsformen wird eine Zusammensetzung in Form eines oralen Direktgranulats bereitgestellt, enthaltend:
Retardpartikel mit einem Magnesium-haltigen Kern enthaltend, oder bestehend aus, Magnesiumoxid, Magnesiumcarbonat, oder Magnesiumchlorid, und einem Überzug welcher zu mindestens 90 Gew.-% aus Glyceroltripalmitat und/oder Glyceroltristearat besteht, bspw. Dynasan^{®}116 oder 118, wobei das Gewichtsverhältnis zwischen Kern und Überzug zwischen 80:20 und 90:10 liegt;
nicht-retardierte Magnesium-Partikel enthaltend, oder bestehend aus, Magnesium-hydrogencitrat und/oder Trimagnesiumdicitrat;
mindestens einen oder mehrere wasserlösliche Hilfsstoffe ausgewählt aus Sorbitol, Xylitol und Mischungen derselben;
mindestens einen oder mehrere speichelanregende Hilfsstoffe, ausgewählt aus Zitronensäure, Mononatriumcitrat und Mischungen derselben;
optional einen oder mehrere schluckbarkeitsfördernde Hilfsstoffe ausgewählt aus Natrium-Carboxymethylcellulose, vollständig vorverkleisterter Stärke (bspw. Maisstärke) und Mischungen derselben;
weiterhin optional Magnesiumstearat als Gleitmittel;
mindestens ein Aroma; sowie mindestens einen Süßstoff ausgewählt aus Sucralose, Aspartam, Acesulfam-K und Mischungen derselben, wobei der Gewichtsanteil der Retardpartikel im oralen Direktgranulat zwischen 30 % und 70 % liegt.

Der Vorteil der erfindungsgemäßen Zusammensetzung in Form eines oralen Direktgranulates gemäß dem ersten Aspekt der Erfindung liegt darin, dass es üblicherweise ohne zusätzliche Flüssigkeit, allein mittels des im Mund vorhandenen Speichel durchfeuchtet bzw. suspendiert und anschließend leicht und angenehm geschluckt werden kann, dabei aber gleichzeitig die kontrollierte Freisetzung des Magnesiums in Form einer retardierten Dosis in Kombination mit einer nicht-retardierten Dosis sicherstellt, selbst wenn in Summe höhere Dosen von z. Bsp. 400 mg Magnesium oder mehr vorliegen. Letzteres gelingt sonst häufig nur in Form voluminöser, schwer schluckbarer Kapsel- oder Tablettenformulierungen.

Diese vorteilhafte Schluckbarkeit wird u.a. durch eine fein aufeinander abgestimmte Selektion und Kombination von Parametern erreicht bzw. unterstützt, wie z. Bsp. die Art und Menge der Hilfsstoffe, welche den Retardpartikeln beigemengt werden, der Gewichtsanteil der Retardpartikel im Direktgranulat, ihre Partikelgröße (sowie die Partikelgröße weiterer Bestandteile des Direktgranulats), die Wahl der Überzugsmaterialien, etc. Vorteilhafte Ausführungsformen sind oben beschrieben.

Die vorteilhafte Verarbeitbarkeit des retardierten Dosis-Anteils in den Retardpartikeln wiederum wird u.a. durch die spezifische Selektion von Magnesium-Kernen mit den beanspruchten Parametern erreicht, welche das Auftragen des Schmelzüberzugs überhaupt erst ermöglichen, insbesondere im Industriemaßstab.

In einem zweiten Aspekt betrifft die vorliegende Erfindung eine Methode gemäß Anspruch 5 zur Charakterisierung eines Magnesium-haltigen Kerns für die Herstellung einer festen, lipid-überzogenen pharmazeutischen oder nutrazeutischen Zusammensetzung in der Wirbelschicht (optional die Herstellung der Retardpartikel der Komponente (a) der Zusammen-setzung gemäß dem ersten Aspekt der Erfindung), wobei der Kern eine Magnesium-verbindung enthält oder aus dieser besteht, und wobei die Methode dadurch gekennzeichnet ist, dass diese mittels einer Vibrationssiebung durchgeführt wird, und dadurch, dass Magnesium-haltige Kerne, welche für die Herstellung der festen, lipid-überzogenen pharmazeutischen oder nutrazeutischen Zusammensetzung in der Wirbelschicht als geeignet charakterisiert werden, bzw. eine Stichprobe hiervon, in diesem Test eine solche Abriebfestigkeit aufweist, dass nach der Vibrationssiebung über einen Zeitraum von 60 Minuten:
- der Feinanteil an Partikeln mit einem Siebdurchmesser von < 200 µm nach einer Siebzeit von 60 Minuten, t₆₀ₘᵢₙ, um maximal 100 Gew.-%, bevorzugt um maximal 50 Gew.-%, weiter bevorzugt um maximal 20 Gew.-%, höher ist gegenüber dem initialen Feinanteil dieses Siebdurchmessers nach einer Siebzeit von 5 Minuten, t₅ₘᵢₙ; und/oder
- der Anteil an Partikeln mit einem Siebdurchmesser von 200 µm ≤ x < 800 µm nach einer Siebzeit von 60 Minuten, t₆₀ₘᵢₙ, um maximal 18 Gew.-%, bevorzugt um maximal 15 Gew.-%, weiter bevorzugt um maximal 12 Gew.-%, niedriger ist gegenüber dem initialen Anteil dieses Siebdurchmessers nach einer Siebzeit von 5 Minuten, t₅ₘᵢₙ.

Es handelt sich somit bei der Methode gemäß dem zweiten Aspekt der Erfindung um eine Eignungs- bzw. Tauglichkeitsprüfung, mit welcher - üblicherweise in stichprobenhafter Form - geprüft werden kann, ob bspw. eine kommerziell erhältliche Rohware Magnesium-haltiger Kerne ausreichend abriebfest ist, um den mechanischen, und ggf. auch den thermischen Belastungen, eines Lipidüberzugsverfahren, insbesondere eines Schmelz-überzugsverfahrens mit Lipiden, in der Wirbelschicht standzuhalten.

Alle Ausführungsformen, die in Verbindung mit der pharmazeutischen oder nutrazeutischen Zusammensetzung gemäß dem ersten Aspekt der Erfindung offenbart sind, einschließlich bevorzugte Ausführungsformen, können gleichermaßen auf die Charakterisierungsmethode gemäß dem zweiten Aspekt der Erfindung angewendet werden. Dies betrifft bspw. die Ausführungen zu den Magnesium-haltigen Kernen sowie zu den aufgetragenen Lipid-Überzügen.

Gemäß der Erfindung wird die Vibrationssiebung mit einer Vibrationsamplitude von ca. 50/min bis ca. 100/min, oder ca. 70/min bis ca. 90/min, oder ca. 75/min bis 85/min, durchgeführt, bspw. bei einer Vibrationsamplitude von ca. 80/min.

Die Vibrationssiebung gemäß der hier beschriebenen Erfindung kann im Grunde mit jedem Siebturm durchgeführt werden, der eine Vibrationssiebung bei diesen Vibrationsamplituden über einen Zeitraum von 60 Minuten gewährleisten kann, bspw. mit einem Siebturm der AS 200 Reihe der Firma Retsch GmbH, wie dem AS 200 basic Modell, welches von den Erfindern vorrangig genutzt wurde.

In einer Ausführungsform wird für die Vibrationssiebung ein Siebturm mit Siebböden von ca. 10-22 cm Siebboden-Durchmesser verwendet. In einer weiteren Ausführungsform wird für die Vibrationssiebung ein Siebturm mit Siebböden von ca. 10-22 cm Siebboden-Durchmesser verwendet, sowie eine zu testende Ausgangsmenge von ca. 50-150 g der Magnesium-haltige Kerne, oder ca. 75-125 g, oder ca. 95-105 g, bspw. ca. 100 g der Magnesium-haltigen Kerne. In einer spezifischen Ausführungsform wird für die Vibrationssiebung ein Siebturm mit Siebböden von ca. 20 cm Siebboden-Durchmesser verwendet wird, sowie eine zu testende Ausgangsmenge der Magnesium-haltigen Kerne von ca. 100 g.

Die Wahl größerer Siebboden-Durchmesser und/oder anderer Mengen der zu testenden Magnesium-haltigen Kerne als hier aufgeführt ist grundsätzlich möglich, ohne damit notwendigerweise vom Geist der vorliegenden Erfindung abzuweichen. Entscheidend ist hierbei jedoch, dass der Fachmann die zu testende Menge der Magnesium-haltigen Kerne gemäß dem Fachmann bekannten Grundsätzen für Siebanalysen an den Durchmesser der verwendeten Siebböden im Siebturm anpasst und diese bspw. nicht überfüllt.

In einer Ausführungsform der Methode wird für die Vibrationssiebung eine Siebhilfe in Form von abriebfesten Kugeln mit einem Durchmesser von ca. 2-6 mm, oder ca. 2-5 mm, oder ca. 2-4 mm verwendet; bspw. ca. 3 mm. In einer weiteren Ausführungsform der Methode wird für die Vibrationssiebung eine Siebhilfe in Form von abriebfesten Kugeln verwendet, welche eine Schüttdichte von ca. 650-850 kg/m³, oder ca. 700-800 kg/m³, aufweisen; wie bspw. ca. 750 kg/m³. In einer der bevorzugten Ausführungs-formen, wird für die Vibrationssiebung eine Siebhilfe in Form von abriebfesten Silicagel-Kugeln mit einem Durchmesser von ca. 2-6 mm oder ca. 2-5 mm, oder ca. 2-4 mm; bspw. ca. 3 mm.

In einer weiteren Ausführungsform der Methode wird für die Vibrationssiebung eine Siebhilfe in Form von abriebfesten Silicagel-Kugeln verwendet, welche eine Schüttdichte von ca. 650-850 kg/m³, oder ca. 700-800 kg/m³ aufweisen, bspw. eine Schüttdichte von ca. 750 kg/m³.

In einer spezifischen Ausführungsform der Methode wird für die Vibrationssiebung eine Siebhilfe in Form von abriebfesten Silicagel-Kugeln mit einem Durchmesser von ca. 2-6 mm, oder ca. 2-5 mm, oder ca. 2-4 mm (bspw. ca. 3 mm), und einer Schüttdichte von ca. 650-850 kg/m³, oder ca. 700-800 kg/m³ verwendet, bspw. mit einer Schüttdichte von ca. 750 kg/m³.

In einer weiteren Ausführungsform der Methode wird für die Vibrationssiebung pro Siebboden je ca. 0,5-2,0 g, oder ca. 0,7-1,5 g, der Siebhilfe verwendet, bspw. ca. 1 g. In einer spezifischen Ausführungsform der Methode wird für die Vibrationssiebung pro Siebboden je ca. 0,5-2,0 g, oder ca. 0,7-1,5 g, bspw. ca. 1 g, einer Siebhilfe in Form von abriebfesten Silicagel-Kugeln mit einem Durchmesser von ca. 2-6 mm, oder ca. 2-5 mm, oder ca. 2-4 mm (bspw. ca. 3 mm), und einer Schüttdichte von ca. 650-850 kg/m³, oder ca. 700-800 kg/m³ verwendet, bspw. mit einer Schüttdichte von ca. 750 kg/m³.

In einer weiteren Ausführungsform der Methode wird für die Vibrationssiebung ein Siebturm mit Siebböden von ca. 10-22 cm Siebboden-Durchmesser verwendet wird, wobei im Siebturm die Siebe mit einem Siebdurchmesser von 800 µm, 600 µm, 500 µm, 400 µm, 300 µm, 200 µm, und 100 µm sowie die Bodenschale verwendet werden.

In einer spezifischen Ausführungsform der Methode wird für die Vibrationssiebung verwendet:
- ein Siebturm mit Sieben mit einem Siebboden-Durchmesser von ca. 10-22 cm und Siebdurchmesser von 800 µm, 600 µm, 500 µm, 400 µm, 300 µm, 200 µm, und 100 µm, sowie die Bodenschale,
- eine zu testende Ausgangsmenge der Magnesium-haltigen Kerne von ca. 50-150 g
- je ca. 0,5-2,0 g Siebhilfe pro Siebboden,
   wobei die abriebfesten Silicagel-Kugeln, welche als Siebhilfe eingesetzt werden, einen Durchmesser von ca. 2-6 mm und eine Schüttdichte von ca. 650-850 kg/m³ aufweisen.

In einer spezifischeren Ausführungsform der Methode wird für die Vibrationssiebung verwendet:
- ein Siebturm mit Sieben mit einem Siebboden-Durchmesser von ca. 10-22 cm und Siebdurchmesser von 800 µm, 600 µm, 500 µm, 400 µm, 300 µm, 200 µm, und 100 µm, sowie die Bodenschale,
- eine zu testende Ausgangsmenge der Magnesium-haltigen Kerne von ca. 75-125 g
- je ca. 0,7-1,5 g Siebhilfe pro Siebboden,
   wobei die abriebfesten Silicagel-Kugeln, welche als Siebhilfe eingesetzt werden, einen Durchmesser von ca. 2-5 mm und eine Schüttdichte von ca. 700-800 kg/m³ aufweisen.

In einer noch spezifischeren Ausführungsform der Methode wird für die Vibrationssiebung verwendet:
- ein Siebturm mit Sieben mit einem Siebboden-Durchmesser von ca. 20 cm und Siebdurchmesser von 800 µm, 600 µm, 500 µm, 400 µm, 300 µm, 200 µm, und 100 µm, sowie die Bodenschale,
- eine zu testende Ausgangsmenge der Magnesium-haltigen Kerne von ca. 100 g,
- je ca. 1 g Siebhilfe pro Siebboden,
   wobei die abriebfesten Silicagel-Kugeln, welche als Siebhilfe eingesetzt werden, einen Durchmesser von ca. 2-4 mm und eine Schüttdichte von ca. 750 kg/m³ aufweisen.

In einer Ausführungsform der Methode ist die Magnesiumverbindung in den Magnesium-haltigen Kernen ausgewählt ist aus Magnesiumoxid (MgO), Magnesiumcarbonat (MgCO₃), Magnesiumchlorid (MgCl₂), Magnesiumhydrogenphosphat (MgHPO₄) und/oder Magnesiumacetat (Mg(CH₃COO)₂). In einer spezifischen Ausführungsform der Methode ist die Magnesiumverbindung in den Magnesium-haltigen Kernen Magnesiumoxid (MgO).

In einer Ausführungsform der Methode gemäß dem zweiten Aspekt der Erfindung, wobei Magnesium-haltige Kerne, welche in diesem Test eine solche Abriebfestigkeit aufweisen, dass nach der Vibrationssiebung über einen Zeitraum von 60 Minuten:
- der Feinanteil an Partikeln mit einem Siebdurchmesser von < 200 µm nach einer Siebzeit von 60 Minuten, t₆₀ₘᵢₙ, um maximal 100 Gew.-%, bevorzugt um maximal 50 Gew.-%, weiter bevorzugt um maximal 20 Gew.-%, höher ist gegenüber dem initialen Feinanteil dieses Siebdurchmessers nach einer Siebzeit von 5 Minuten, t₅ₘᵢₙ; und/oder
- der Anteil an Partikeln mit einem Siebdurchmesser von 200 µm ≤ x < 800 µm nach einer Siebzeit von 60 Minuten, t₆₀ₘᵢₙ, um maximal 18 Gew.-%, bevorzugt um maximal 15 Gew.-%, weiter bevorzugt um maximal 12 Gew.-%, niedriger ist gegenüber dem initialen Anteil dieses Siebdurchmessers nach einer Siebzeit von 5 Minuten, t₅ₘᵢₙ.
insbesondere für die Herstellung der festen, lipid-überzogenen pharmazeutischen oder nutrazeutischen Zusammensetzung mittels eines Schmelzbeschichtungsverfahrens in der Wirbelschicht geeignet sind.

In einem dritten Aspekt betrifft die vorliegende Erfindung ein Verfahren gemäß Anspruch 11 zur Herstellung einer festen pharmazeutischen oder nutrazeutischen Zusammensetzung gemäß dem oben beschriebenen ersten Aspekt der Erfindung, wobei das Verfahren die folgenden Schritte enthält bzw. umfasst:
(i) Bereitstellen eines Magnesium-haltigen Kerns, wobei der Kern eine Magnesium-verbindung enthält oder aus dieser besteht;
(ii) Bereitstellen eines geschmolzenen Überzugsmaterials enthaltend ein Lipid;
(iii) Fluidisieren der Magnesium-haltigen Kerne;
(iv) Besprühen der fluidisierten Magnesium-haltigen Kerne mit dem geschmolzenen Überzugsmaterial;
(v) Abkühlen der überzogenen Magnesium-haltigen Kerne, so dass das Lipid erstarrt und Retardpartikel gemäß Komponente (a) erhalten werden, welche das enthaltene Magnesium in retardierter Weise freisetzen; und
(vi) Mischen der so als Komponente (a) erhaltenen Retardpartikel mit den folgenden weiteren Komponenten:
   (b) nicht-retardierte Partikel mit einem zweiten Magnesium-haltigen Kern, wobei der Partikel eine Magnesiumverbindung enthält oder aus dieser besteht, und wobei die Partikel das enthaltene Magnesium in nicht-retardierter Weise freisetzen;
   (c) einen oder mehrere wasserlösliche Hilfsstoffe ausgewählt aus der Gruppe der Zucker, Zuckeralkohole und Oligosaccharide; und
   (d) optional weiteren Hilfsstoffen;
dadurch gekennzeichnet, dass mindestens die Magnesium-haltigen Kerne der Retard-partikel der Komponente (a), bzw. eine Stichprobe hiervon, eine solche Abriebfestigkeit aufweisen, dass nach einer Vibrationssiebung über einen Zeitraum von 60 Minuten:
- der Feinanteil an Partikeln mit einem Siebdurchmesser von < 200 µm nach einer Siebzeit von 60 Minuten, t₆₀ₘᵢₙ, um maximal 100 Gew.-% höher ist gegenüber dem initialen Feinanteil dieses Siebdurchmessers nach einer Siebzeit von 5 Minuten, t₅ₘᵢₙ; und/oder
- der Anteil an Partikeln mit einem Siebdurchmesser von 200 µm ≤ x < 800 µm nach einer Siebzeit von 60 Minuten, t₆₀ₘᵢₙ, um maximal 18 Gew.-% niedriger ist gegenüber dem initialen Anteil dieses Siebdurchmessers nach einer Siebzeit von 5 Minuten, t₅ₘᵢₙ.

In anderen Worten, in Schritt (i) des Herstellungsverfahrens gemäß dem dritten Aspekt der Erfindung werden nur jene Magnesium-haltigen Kerne, oder Chargen hiervon, bereitgestellt, welche mittels der Methode gemäß dem zweiten Aspekt der Erfindung als für das Herstellungsverfahren geeignet charakterisiert wurden; bzw. die Methode zur Charakterisierung der Magnesium-haltigen Kerne gemäß dem zweiten Aspekt der Erfindung kann dem Schritt (i) des Herstellungsverfahrens vorgeschaltet sein, um so nur solche Magnesium-haltigen Kerne, oder Chargen hiervon, für das Lipidüberzugs-verfahren, und insbesondere für ein Schmelzüberzugsverfahren mit Lipiden, in der Wirbelschicht auszuwählen, welche die erforderliche, oben an beschriebene Abrieb-festigkeit hierfür mitbringen. In diesem Zusammenhang ist jedoch noch einmal anzumerken, dass üblicherweise eine Charge der zu überziehenden Magnesium-haltigen Kerne nur stichprobenartig auf die geforderten Abriebs-Parameter geprüft werden muss, und dass somit nicht jedem einzelnen Herstellungsvorgang gemäß dem dritten Aspekt der Erfindung auch notwendigerweise die Charakterisierungsmethode mittels Vibrationssiebung gemäß dem zweiten Aspekt vorangestellt sein muss.

Alle Ausführungsformen, die in Verbindung mit der pharmazeutischen oder nutrazeutischen Zusammensetzung gemäß dem ersten Aspekt der Erfindung oder der Charakterisierungsmethode gemäß dem zweiten Aspekt der Erfindung offenbart sind, einschließlich bevorzugte Ausführungsformen, können gleichermaßen auf das Herstellverfahren gemäß dem dritten Aspekt der Erfindung angewendet werden.

So weisen bspw. in einer spezifischen Ausführungsform mindestens die im Schritt (i) bereitgestellten Magnesium-haltigen Kerne der Retardpartikel der Komponente (a), bzw. eine Stichprobe hiervon, eine solche Abriebfestigkeit auf, dass nach einer Vibrationssiebung über einen Zeitraum von 60 Minuten der Feinanteil an Partikeln mit einem Siebdurchmesser von < 200 µm nach einer Siebzeit von 60 Minuten, t₆₀ₘᵢₙ, um maximal 50 Gew.-%, bevorzugt um maximal 20 Gew.-%, höher ist gegenüber dem initialen Feinanteil dieses Siebdurchmessers nach einer Siebzeit von 5 Minuten, t₅ₘᵢₙ; und/oder der Anteil an Partikeln mit einem Siebdurchmesser von 200 µm ≤ x < 800 µm nach einer Siebzeit von 60 Minuten, t₆₀ₘᵢₙ, um maximal 15 Gew.-%, bevorzugt um maximal 12 Gew.-%, niedriger ist gegenüber dem initialen Anteil dieses Siebdurchmessers nach einer Siebzeit von 5 Minuten, t₅ₘᵢₙ.

In einer weiteren Ausführungsform weisen mindestens die im Schritt (i) bereitgestellten Magnesium-haltigen Kerne der Retardpartikel der Komponente (a), bzw. eine Stichprobe hiervon, eine solche Abriebfestigkeit auf, dass nach einer Vibrationssiebung über einen Zeitraum von 60 Minuten der Anteil an Partikeln mit einem Siebdurchmesser von < 300 µm nach einer Siebzeit von 60 Minuten, t₆₀ₘᵢₙ, um maximal 55 Gew.-%, bevorzugt um maximal 35 Gew.-%, weiter bevorzugt um maximal 15 Gew.-%, höher ist gegenüber dem initialen Anteil dieses Siebdurchmessers nach einer Siebzeit von 5 Minuten, t₅ₘᵢₙ; und/oder der Anteil an Partikeln mit einem Siebdurchmesser von 300 µm ≤ x < 800 µm nach einer Siebzeit von 60 Minuten, t₆₀ₘᵢₙ, um maximal 30 Gew.-%, bevorzugt um maximal 27 Gew.-%, weiter bevorzugt um maximal 24 Gew.-%, niedriger ist gegenüber dem initialen Anteil dieses Siebdurchmessers nach einer Siebzeit von 5 Minuten, t₅ₘᵢₙ.

Die Vibrationssiebung kann wie oben an im zweiten Aspekt der Erfindung bereits beschrieben durchgeführt werden: eine zu siebende Menge von ca. 50-150 g der Magnesium-haltige Kernen, oder ca. 75-125 g, oder ca. 95-105 g, bspw. ca. 100 g der Magnesium-haltigen Kerne wird auf den Siebturm gegeben (auf das oberste Sieb des Turmes, bspw. ein 800 µm Sieb), zusammen mit je ca. 0,5-2,0 g, oder ca. 0,7-1,5 g, bspw. ca. 1 g Siebhilfe pro Siebboden (hier z. Bsp. abriebfeste ca. 2-6 mm, oder ca. 2-5 mm, oder ca. 2-4 mm große Silicagel-Kugeln (bspw. ca. 3 mm) mit einer Schüttdichte von ca. 650-850 kg/m³, oder ca. 70-800 kg/m³, wie bspw. ca. 750 kg/m³) und für 60 min bei einer Vibrationsamplitude von ca. 50/min bis ca. 100/min, oder ca. 70/min bis ca. 90/min, oder ca. 75/min bis 85/min, bspw. ca. 80/min gesiebt und auf diese Weise mechanisch gestresst. In einer der vorteilhaften Ausführungsformen ist - wie oben beschrieben - die Siebanalyse (also die Charakterisierungsmethode gemäß dem zweiten Aspekt) dem Verfahren gemäß dem dritten Aspekt der Erfindung vorangestellt, um so vorab die Tauglichkeit der Magnesium-haltigen Kerne - oder in anderen Worten deren Eignung - für ein Lipidüberzugsverfahren in der Wirbelschicht, bspw. insbesondere für ein Schmelzbeschichtungsverfahren (Hot-Melt Coating) in der Wirbelschicht zu prüfen.

Kerne, welche zu viel Abrieb aufweisen (sprich mehr als im Rahmen der Erfindung zulässig), eignen sich nicht für ein Hot-Melt Coating in der Wirbelschicht, insbesondere nicht im Industriemaßstab. Während früherer Versuche, ein schmelzüberzogenes Magnesium-Direktgranulat herzustellen, hatten die Erfinder festgestellt, dass der starke Abrieb einiger kommerziell erhältlicher Magnesium-Qualitäten, die Filter zu stark verstopfte weit bevor der Schmelzüberzugsprozess ausreichend weit vorangeschritten war; ein Problem, dass sich auch nicht mit den üblichen automatisierten Filterabschlägen der Wirbelschichtgeräte beheben ließ. Die somit in diesen Versuchen benötigten häufigen Filterwechsel, bzw. Filterreinigungsschritte, machten die früheren Verfahren ungeeignet für den Industriemaßstab. Zudem waren, wie oben erwähnt, die entstehenden Auflagerungen von verkrustetem Kern-Abrieb und Sprühmaterial auf den Innenwänden des Wirbelschicht-Geräts so ausgeprägt und fest, dass stets sehr zeitaufwändige Reinigungsschritte nötig waren.

Das in den Schritten (i)-(v) beschriebene Verfahren, und optional auch der Mischungsschritt (vi), kann in Wirbelschichtgeräten (sog. fluid-bed coater oder air-flow-bed coater) jeglicher Art durchgeführt werden, sofern die Geräte eine ausreichende Temperierung erlauben, um eine vorzeitige Erstarrung des geschmolzenen Überzugsmaterials zu vermeiden. Temperatur und Sprührate der Schmelze sollte in jedem Fall so angepasst sein, dass eine gleichmäßige Befilmung der einzelnen Kern-Partikel gewährleistet ist, da größere Agglomerate, in denen bspw. zwei oder mehr Kern-Partikel miteinander ,verklebt' sind, für ein gutes Mundgefühl des Direktgranulates nicht erwünscht sind.

Wie oben erwähnt, enthält der Überzug der Retardpartikel in einer der bevorzugten Ausführungsformen ein Lipid mit einem Schmelzpunkt von mindestens 50 °C, oder besteht im Wesentlichen hieraus; bevorzugt ein Lipid mit einem Schmelzpunkt von mindestens 60 °C. Um einen gleichmäßigen Auftrag des geschmolzenen Überzugs-materials zu gewährleisten, liegt in einer Ausführungsform des Verfahrens die Prozesslufttemperatur in den Schritten (iii) und/oder (iv) zwischen 20 und 60 °C, oder zwischen 20 und 50 °C, oder zwischen 20 und 45 °C; bspw. bei ca. 25 °C oder 35 °C.

In einer Ausführungsform des Verfahrens wird das in Schritt (ii) bereitgestellte geschmolzene Überzugsmaterial im Schritt (iv) bei einer Schmelztemperatur zwischen 70 und 120 °C, oder zwischen 75 und 110 °C, oder zwischen 80 und 100 °C versprüht; bspw. bei ca. 90 bis 100 °C. So liegt bspw. der Schmelzpunkt von Glyceroltristearat (z. Bsp. Dynasan^{®}118) bei ca. 70-73 °C; daher sollte die Temperatur der zu sprühenden Schmelze hierbei einige Grad darüber liegen; z. Bsp. bei 90 °C.

In einer Ausführungsform des Verfahrens wird im Schritt (iv) eine Sprühlufttemperatur zwischen 70 und 130 °C, oder zwischen 75 und 125 °C, oder zwischen 80 und 120 °C verwendet; bspw. ca. 100 °C oder 120 °C. Des Weiteren wird in einer Ausführungsform des Verfahrens im Schritt (iv) ein Sprühdruck zwischen 0,5 und 1,5 bar gewählt, oder zwischen 0,6 und 1,3 bar, oder zwischen 0,7 und 1,2 bar; bspw. bei 0,8 bis 1,2 bar, 0,8 bis 0,9 bar oder 1,0 bis 1,2 bar.

In einer Ausführungsform des Verfahrens liegt die Sprühmenge des geschmolzenen Überzugsmaterials pro Kilogramm zu überziehendem Kern-Material im Schritt (iv) zwischen 2,0 und 10,0 g/kg/min, oder zwischen 2,5 und 9,0 g/kg/min, oder zwischen 3,0 und 8,0 g/kg/min; bspw. bei ca. 3,3 g/kg/min. In einer spezifischen Ausführungsform des Verfahrens wird dieses - und insbesondere die Überzugsschritte (iii) bis (v) - in einem Wirbelschichtgerät im Labor-Maßstab (z. Bsp. in einem Ventilus^{®}V-2.5) durchgeführt, und die Sprühmenge des geschmolzenen Überzugsmaterials im Schritt (iv) liegt zwischen 5,0 und 8,0 g/min, oder zwischen 5,5 und 7,5 g/min, oder zwischen 6,0 und 7,0 g/min; bspw. bei ca. 6,5 g/min.

Die gemäß Verfahrensschritt (vi) neben den Retardpartikeln der Komponente (a) vorliegenden Komponenten (c) bis (d), optional (b) bis (d), werden üblicherweise zuerst homogen miteinander vermischt, bevor sie anschließend mit den Retardpartikeln vermengt werden. Wie erwähnt können optional einzelne oder alle der Substanzen oder Hilfsstoffe gemäß Komponenten (b), (c) und/oder (d) vor dem Vermengen mit den Retardpartikeln granuliert bzw. aggregiert werden.

In einer Ausführungsform des Verfahrens werden in einem weiteren Schritt, im Anschluss an Schritt (vi), Einzeldosen der Zusammensetzung in Stickpacks, Sachets, Glasampullen oder Plastikampullen verpackt.

In einem weiteren Aspekt der Erfindung betrifft die vorliegende Erfindung eine feste pharmazeutische oder nutrazeutische Zusammensetzung gemäß Anspruch 12 in Form eines oralen Direktgranulats mit mindestens dualer Wirkstofffreisetzung, welche(s) mittels des Verfahrens gemäß dem dritten Aspekt der Erfindung hergestellt wird.

### BEISPIELE

### Beispiel 1: Vergleich der Eigenschaften verschiedener MgO-Kerne

Granulierte Magnesiumoxid (MgO) Partikel verschiedener Anbieter wurde unter anderem auf ihre Partikelgröße und ihre Abriebfestigkeit hin untersucht, um zu prüfen, welche Ware sich zur Verwendung als Kern der Retardpartikel in einem Hotmelt-Coating Prozess eignet; darunter u.a. trockenkompaktierte Rohwaren wie das'Heavy Magnesium Oxide EP' von Kyowa Chemical Industry Co., Ltd Japan (nachstehend als 'MgO 1' bezeichnet) und MagGran^{®} MO der Magnesia GmbH Deutschland (nachstehend ,MgO 2').

Um den Abrieb der MgO-Kerne während des Hot-Melt Coatings in der Wirbelschicht zu simulieren, wurden diese für insgesamt 60 Minuten lang auf einem Siebturm (hier bspw. ein Retsch AS 200 basic, Retsch GmbH, Haan) mit Siebdurchmesser von ca. 20 cm mechanisch gestresst, wobei zusätzlich eine Siebhilfe in Form von abriebfesten Silicagel-Kugeln verwendet wurde, welche einen Durchmesser von ca. 2-5 mm (hier bspw. ca. 3 mm) und eine Schüttdichte von ca. 650-850 kg/m³ (hier ungefähr 750 kg/m³) aufweisen. Hierbei wurde zunächst eine Probenmenge von ca. 100 g der MgO-Kerne auf das oberste Sieb (800 µm) gegeben und dazu je 1 g Siebhilfe pro Siebboden. Für die Siebung wurde eine Vibrationsamplitude von 80/min verwendet. Alle 5 Minuten wurden die einzelnen Siebe (800 µm, 600 µm, 500 µm, 400 µm, 300 µm, 200 µm, 100 µm und Rückstandsschale) sowie die darauf befindliche Menge an MgO-Kernen gewogen. Die Daten der beiden Siebanalysen sind in Tabellen 1a (MgO 1) und 1b (MgO 2) sowie in den Abb. 1a und 1b dargestellt.

**Tabelle 1a: Siebanalyse der MgO 1-Kerne**

| **Sieb [µm]** | **Siebdauer [min]** | | | | | | | | | | | |
|---|---|---|---|---|---|---|---|---|---|---|---|---|
| | **5** | **10** | **15** | **20** | **25** | **30** | **35** | **40** | **45** | **50** | **55** | **60** |
| **800** | 0,0 | 0,0 | 0,0 | 0,0 | 0,0 | 0,0 | 0,0 | 0,0 | 0,0 | 0,0 | 0,0 | 0,0 |
| **600** | 0,0 | 0,0 | 0,0 | 0,0 | 0,0 | 0,0 | 0,0 | 0,0 | 0,0 | 0,0 | 0,0 | 0,0 |
| **500** | 0,1 | 0,0 | 0,0 | 0,0 | 0,0 | 0,0 | 0,0 | 0,0 | 0,0 | 0,0 | 0,0 | 0,1 |
| **400** | 6,4 | 5,4 | 4,8 | 4,4 | 4,1 | 3,8 | 3,5 | 3,2 | 3,0 | 2,8 | 2,6 | 2,4 |
| **300** | 24,7 | 24,2 | 24,0 | 23,8 | 23,6 | 23,4 | 23,2 | 23,1 | 23,0 | 22,9 | 22,8 | 22,6 |
| **200** | 35,0 | 35,0 | 34,8 | 34,7 | 34,6 | 34,5 | 34,5 | 34,4 | 34,4 | 34,4 | 34,4 | 34,3 |
| **100** | 33,3 | 34,6 | 35,4 | 36,1 | 36,6 | 36,9 | 37,4 | 37,6 | 37,8 | 38,1 | 38,3 | 38,5 |
| **<100** | 0,9 | 1,1 | 1,2 | 1,3 | 1,5 | 1,6 | 1,9 | 2,0 | 2,1 | 2,2 | 2,3 | 2,4 |

**Tabelle 1b: Siebanalyse der MgO 2-Kerne**

| **Sieb [µm]** | **Siebdauer [min]** | | | | | | | | | | | |
|---|---|---|---|---|---|---|---|---|---|---|---|---|
| | **5** | **10** | **15** | **20** | **25** | **30** | **35** | **40** | **45** | **50** | **55** | **60** |
| **800** | 0,1 | 0,1 | 0,1 | 0,1 | 0,0 | 0,1 | 0,1 | 0,1 | 0,1 | 0,1 | 0,0 | 0,1 |
| **600** | 14,6 | 12,3 | 11,0 | 9,8 | 9,0 | 8,3 | 7,7 | 7,2 | 6,6 | 6,1 | 5,7 | 5,3 |
| **500** | 18,1 | 17,5 | 16,7 | 15,8 | 15,2 | 14,5 | 13,8 | 13,2 | 12,6 | 11,9 | 11,4 | 10,7 |
| **400** | 15,7 | 15,4 | 14,9 | 14,3 | 13,8 | 13,3 | 12,8 | 12,2 | 11,7 | 11,1 | 10,6 | 10,1 |
| **300** | 20,9 | 21,0 | 20,9 | 20,6 | 20,3 | 20,1 | 19,8 | 19,4 | 19,1 | 18,6 | 18,2 | 17,8 |
| **200** | 21,6 | 22,9 | 23,7 | 24,6 | 25,1 | 25,6 | 26,1 | 26,6 | 27,0 | 27,4 | 27,8 | 28,2 |
| **100** | 5,6 | 6,8 | 7,9 | 8,9 | 9,7 | 10,4 | 11,2 | 11,9 | 12,6 | 13,3 | 14,0 | 14,7 |
| **<100** | 3,0 | 3,9 | 4,9 | 5,9 | 6,8 | 7,7 | 8,6 | 9,5 | 10,5 | 11,4 | 12,3 | 13,2 |

Alternativ hierzu, wurde die Abriebfestigkeit der MgO-Kerne auch noch mittels eines Einsaugversuches bestimmt bei dem 400 g der MgO-Kerne mittels Vakuums in ein Wirbelschichtgerät im Labor-Maßstab eingesaugt, und in diesem anschließend für ca. 5 min bei einer Temperatur von 25°C und einer Luftmenge von 35 m³/h trocken verwirbelt wurden. In diesem Beispiel wurde hierfür ein Ventilus^{®}V-2.5 Gerät verwendet (Romaco Innojet, Steinen, Deutschland).

Zusätzlich hierzu wurde in beiden Versuchen (Siebanalyse und Einsaugversuch) die Partikelgröße der verwendeten Kerne mittels dynamischer Bildanalyse bestimmt, in einem Camsizer^{®} XT Gerät, ausgestattet mit der X-Jet Plug-in Kartusche sowie der dazugehörigen Analyse-Software (Retsch Technology GmbH, Haan, Deutschland). Die entsprechenden Daten - jeweils aus der Volumenverteilung bestimmt - sind in Tabelle 2 gelistet:

**Tabelle 2: Partikelgrößen zweier verschiedener Rohwaren von MgO Kernen**

| | **MgO 1** | | | **MgO 2** | | |
|---|---|---|---|---|---|---|
| | **a** | **b** | **c** | **a** | **b** | **c** |
| **D10** | 88,0 | 99,9 | 75,7 | 53,2 | 28,0 | 18,3 |
| **D50** | 223,4 | 225,4 | 222,3 | 354,7 | 264,5 | 294,4 |
| **Mittelwert** | 234,4 | 234,7 | 231,4 | 358,2 | 285,0 | 307,3 |
| **D90** | 399,4 | 390,2 | 397,6 | 644,3 | 588,3 | 630,4 |
| **Streuung** | 118,7 | 111,9 | 119,1 | 211,4 | 204,8 | 228,5 |
| **(D90-D10)/D50** | 1,39 | --- | --- | 1,67 | --- | --- |

| | | | | | | |
|---|---|---|---|---|---|---|
| a) ungestresst (Rohware der MgO-Kerne) b) Abrieb nach 60 min Siebturm mit Siebhilfe (2-5 mm Silicagel; ρ = 750 kg/m³) c) Abrieb nach 5 min Ventilus Einsaugversuch und Verwirbelung | | | | | | |

Wie sich aus den Camsizer-Daten ablesen lässt, ist die MgO 2 Rohware der Magnesium-oxid-haltigen Kerne deutlich anfälliger für Abrieb und/oder Bruch der Kerne als die MgO 1 Rohware, und zeigt bei allen vier Parametern (D10, D90, Median D50 und Mittelwert der volumenbezogenen Partikelgrößenverteilung) eine Verkleinerung der Werte. Im Gegensatz hierzu bleiben die Werte der MgO 1-Rohware im Rahmen der Messstreuung überwiegend stabil, was auf eine eher geringe Neigung zu Staub-Abrieb oder Bruch der Kerne hinweist.

Dieselbe Tendenz ist auch aus den Abb. 1a und 1b ersichtlich: während der Feinanteil (<200 µm in Abb. 1a, und <300 µm in Abb. 1b) für die MgO 2 Kerne stetig und annähernd linear steigt und in Konsequenz die Masse an Kernen in den respektiven Partikelgrößen-Klassen 200-800 µm bzw. 300-800 µm stetig sinkt über den Verlauf der 60 min Siebdauer, ist für die MgO 1 Kerne nach einem initialen Abrieb während der ersten 15-20 min anschließend nur noch sehr moderater Abrieb zu verzeichnen. Jedoch selbst in diesen ersten 15-20 min ist der Abrieb für die MgO 1 Rohware deutlich geringer als für die MgO 2 Rohware.

In der Konsequenz ermöglicht die vorteilhafte erfindungsgemäße Abriebfestigkeit der MgO 1 Kerne ein Schmelzbeschichtungsverfahren, welches zuvor mit diversen anderen kommerziell erhältlichen MgO Kernen nicht möglich war, bzw. nicht in reproduzierbarer Weise und nicht im industriellen Maßstab (bspw. ≥50 kg pro Charge) möglich war.

### Beispiel 2: Herstellung überzogener MgO-Retardpartikel im Labormaßstab

MgO 1 Kerne wie in Beispiel 1 (hier 0,8 kg) wurden in einem Wirbelschichtgerät (hier ein Ventilus V 2.5 ausgestattet mit einem Romaco Innojet Hot-Melt-Gerät IHD 1; Romaco Innojet, Steinen, Deutschland) im Hotmelt-Coating Verfahren mit geschmolzenem Glyceroltristearat (hier Dynasan^{®} 118) überzogen. Die Temperatur der Schmelze lag bei 90-100 °C, der Sprühdruck bei ca. 0,8-0,9 bar, die Sprührate ca. 6,5 g/min, und die Sprühluft-Temperatur bei ca. 100 °C.

Auf diese Weise wurden Überzugslevel von 30 Gew.-%, 20 Gew.-%, 15 Gew.-%, und 10 Gew.-% bezogen auf das Gewicht der überzogenen MgO-Retardpartikel hergestellt (d.h. 42,9 Gew.-%, 25,0 Gew.-%, 17,6 Gew.-%, und 11,1 Gew.-% bezogen auf das Ursprungsgewicht der nicht-überzogenen MgO-Kerne). Eine Menge von ca. 921 mg Retardpartikel mit einem Überzugslevel von 10 Gew.-% enthalten demnach ca. 829 mg MgO (entsprechend ca. 500 mg Mg) und ca. 92 mg des Dynasan^{®}118.

**Tabelle** 3 stellt die Partikelgrößen der Rohware (nicht-überzogene Kerne) im Vergleich mit der überzogenen Charge mit 10 % Überzugslevel dar, und deutet auf einen ungefähr 20 µm dicken Überzug hin. Zudem ist die Partikelgrößenverteilung der überzogenen Kerne etwas enger, wie der etwas geringere Quotient (D90-10)/D50 andeutet.

**Tabelle 3: Partikelgrößen von MgO 1 Kernen (nicht überzogen vs. Überzugslevel 10 %)**

| | **MgO 1** | **MgO 1 90:10** |
|---|---|---|
| **D10** | 95,5 | 136,8 |
| **D50** | 206,1 | 250,3 |
| **Mittelwert** | 217,0 | 259,5 |
| **D90** | 359,5 | 401,5 |
| **Streuung** | 101,2 | 105,8 |
| **(D90-D10)/D50** | 1,28 | 1,06 |

### Beispiel 3: Herstellung überzogener MgO-Retardpartikel im Industriemaßstab

MgO 1 Kerne wie in Beispiel 1 (hier 76,5 kg) wurden in einem Wirbelschichtgerät (hier ein Ventilus V 100 ausgestattet mit einem Romaco Innojet Hot-Melt-Gerät IHD 1; Romaco Innojet, Steinen, Deutschland) im Hotmelt-Coating Verfahren mit geschmolzenem Glyceroltristearat (hier Dynasan^{®} 118) überzogen. Die Temperatur der Schmelze lag bei 90-100 °C, der Sprühdruck bei ca. 1,0-1,2 bar, die Sprührate ca. 250 g/min, und die Sprühluft-Temperatur bei ca. 120 °C.

Auf diese Weise wurden Überzugslevel von 30 Gew.-%, 20 Gew.-%, 15 Gew.-%, und 10 Gew.-% bezogen auf das Gewicht der überzogenen MgO-Retardpartikel hergestellt (d.h. 42,9 Gew.-%, 25,0 Gew.-%, 17,6 Gew.-%, und 11,1 Gew.-% bezogen auf das Ursprungsgewicht der nicht-überzogenen MgO-Kerne).

### Beispiel 4: Herstellung oraler Direktgranulat-Formulierungen mit MgO Retardpartikeln

Die in Beispiel 2 erhaltenen MgO-Retardpartikel mit einem Überzugslevel von 15 Gew.-% oder 10 Gew.-% wurden mit verschiedenen Hilfsstoffmischungen versetzt (Tabelle 4 und Tabelle 5). Die Hilfsstoffmischungen wurden zunächst separat vermengt und anschließend den MgO-Retardpartikeln zugemischt. Der Gewichtsanteil der MgO-Retardpartikel am Gesamtgewicht der finalen Mischung, dem finalen oralen Direktgranulat, beträgt hierbei ca. 57 Gew.-% für die Mischung der Tabelle 4, und ca. 34 Gew.-% für die Mischung der Tabelle 5.

Die oralen Direktgranulate ODG 1-3 enthalten jeweils ca. 500 mg Magnesiumoxid in Retardpartikeln, 7,85 mg Trimagnesiumdicitrat in Form von nicht-retardierten Partikeln, sowie 10 µg Vit. D3 und 20 µg Vit. B12. Die drei Direktgranulate unterscheiden sich vorrangig in ihren Geschmacksgebern wie den Aromen, Zuckeralkoholen und/oder Süßstoffen.

Das orale Direktgranulat ODG 4 enthält ca. 350 mg Magnesiumoxid in Retardpartikeln, 42,15 mg Magnesiumhydrogencitrat und 7,85 mg Trimagnesiumdicitrat (beide in Form von nicht-retardierten Partikeln), sowie 1,1 mg Vit. B1, 1,4 mg Vit. B2 1,4 mg Vit. B6 und 2,5 µg Vit. B12.

**Tabelle 4: Zusammensetzung der zur Komponente (a) zugemischten Komponenten (Angaben in %)**

| **Bestandteile** | **ODG 1 MgO 1 85:15** | **ODG 2 MgO 1 85:15** | **ODG 3 MgO 1 90:10** |
|---|---|---|---|
| Sorbitol | 35,98 | 35,98 | 37,26 |
| Xylitol | 27,60 | 27,60 | 19,15 |
| Mononatriumcitrat (wasserfrei) | 7,59 | 7,59 | 14,73 |
| Calcium-Brausebasis | 6,90 | 6,90 | 7,37 |
| Trimagnesiumdicitrat (wasserfrei) | 6,90 | 6,90 | 7,37 |
| Zitronensäure | 4,83 | 4,83 | 2,95 |
| Cyanocobalamin (Vit. B12) 0,1% | 3,17 | 3,17 | 3,39 |
| Carmellose Natrium (Na-CMC) | 2,76 | 2,76 | 2,95 |
| Aroma Zitrone | 2,76 | --- | 2,95 |
| Aroma Multifrucht | --- | 2,76 | --- |
| Cholecalciferol (Vit. D3) | 0,75 | 0,75 | 0,80 |
| Magnesiumstearat | 0,55 | 0,55 | 0,59 |
| Aspartam | 0,21 | 0,21 | 0,44 |
| Acesulfam-K | --- | --- | 0,07 |

**Tabelle 5: Zusammensetzung der zur Komponente (a) zugemischten Komponenten (Angaben in %)**

| **Bestandteile** | **ODG 4 MgO 1 90:10** |
|---|---|
| Magnesiumhydrogencitrat | 39,51 |
| Sorbitol | 43,81 |
| Xylitol | 7,97 |
| Trimagnesiumdicitrat (wasserfrei) | 3,98 |
| Carmellose Natrium | 1,59 |
| Aroma Orange | 1,60 |
| Pyridoxin-HCl (Vit. B1) | 0,47 |
| Riboflavin (Vit. B2) | 0,38 |
| Thiaminnitrat (Vit. B6) | 0,37 |
| Cyanocobalamin (Vit. B12) 0,1% | 0,23 |
| Sucralose | 0,09 |

### Beispiel 5: Freisetzungsverhalten in 0.1 N Salzsäure

Das Freisetzungsverhalten von Magnesium aus den schmelzüberzogenen Retardpartikeln des Beispiels 2 mit einem Überzugslevel von 10 Gew.-% (90:10) bzw. 15 Gew.-% (85:15) sowie den damit formulierten erfindungsgemäßen oralen Direktgranulaten aus Beispiel 4 wurde in einer arzneibuchgemäßen Blattrührer-Freisetzungsapparatur (USP Dissolution Apparatus 2) bei 37 °C in 900 mL 0.1 N Salzsäure (HCl) bestimmt. Die Rührgeschwindigkeit betrug 75 UPM. Alle Versuche wurden mindestens sechs Mal wiederholt und Mittelwerte sowie Standard-abweichungen berechnet.

Wie aus Abb. 2 ersichtlich, ist die Freisetzung aus schmelzüberzogenen MgO 1-Kernen bei einem Überzugslevel von 15 Gew.-% ausreichend retardiert, um eine stabile Magnesium-versorgung über den gesamten Tag zu gewährleisten. Zudem sind die Freisetzungsprofile der beiden erfindungsgemäßen Zusammensetzungen ODG 1 und ODG 2 dem Freisetzungsprofil der bloßen schmelzüberzogenen MgO 1-Kernen sehr ähnlich, was darauf hinweist, dass die übrigen Bestandteile des Direktgranulates die Freisetzung aus den Retardpartikeln nicht negativ beeinflussen.

Wie aus Abb. 3 ersichtlich, ist die Freisetzung aus schmelzüberzogenen MgO 1-Kernen bei einem Überzugslevel von nur 10 Gew.-% wie erwartet etwas schneller als bei einem Überzugslevel von 15 Gew.-%; sie kann also mit dem Überzugslevel eingestellt werden. Beide sind jedoch ausreichend retardiert, um eine stabile Magnesiumversorgung über den gesamten Tag zu gewährleisten.

Abb. 4 zeigt die Freisetzung aus schmelzüberzogenen MgO 1-Kernen bei einem Überzugslevel von 10 Gew.-% für ein orales Direktgranulat welches ca. 50 mg nicht-retardiertes Magnesium und ca. 350 mg retardiertes Magnesium in Form der schmelzüberzogenen MgO 1-Kerne enthält (ODG 4), sowie im Vergleich hierzu ein orales Direktgranulat welches nur ca. 8 mg nicht-retardiertes Magnesium und ca. 500 mg retardiertes Magnesium in Form der schmelzüberzogenen MgO 1-Kerne enthält (ODG 3). Wie aus Abb. 4 ersichtlich, ist die Magnesium-Freisetzung des ODG 4 aufgrund des darin enthaltenen höheren nicht-retardierten Anteils schneller als für ODG 3 mit überwiegend retardierten Magnesium-Anteilen. Dies zeigt, dass die Magnesium-Freisetzung nicht nur mittels des Überzugslevels angepasst werden kann, sondern auch durch die Wahl des Anteils nicht-retardiertes versus retardiertes Magnesium.

## Patentansprüche

1. Feste pharmazeutische oder nutrazeutische Zusammensetzung in Form eines oralen Direktgranulats mit mindestens dualer Wirkstofffreisetzung, enthaltend folgende Komponenten:
- überzogene Retardpartikel mit einem Magnesium-haltigen Kern und einem Lipid-Überzug, wobei der Kern eine Magnesiumverbindung enthält oder aus dieser besteht, und wobei die Retardpartikel das enthaltene Magnesium in retardierter Weise freisetzen;
- nicht-retardierte Magnesium-haltige Partikel, wobei die Partikel eine Magnesiumverbindung enthalten oder aus dieser bestehen, und wobei die Partikel das enthaltene Magnesium in nicht-retardierter Weise freisetzen;
- einen oder mehrere wasserlösliche Hilfsstoffe ausgewählt aus der Gruppe der Zucker, Zuckeralkohole und Oligosaccharide; und
- optional einen oder mehrere weitere Hilfsstoffe;
**dadurch gekennzeichnet, dass** die Retardpartikel in einem Schmelzüberzugsprozess in der Wirbelschicht hergestellt sind, und dass mindestens die Magnesium-haltigen Kerne der Retardpartikel der Komponente (a), bzw. eine Stichprobe hiervon, eine solche Abrieb-festigkeit aufweisen, dass nach einer Vibrationssiebung über einen Zeitraum von 60 Minuten:
- der Feinanteil an Partikeln mit einem Siebdurchmesser von < 200 µm nach einer Siebzeit von 60 Minuten, t₆₀ₘᵢₙ, um maximal 100 Gew.-%, bevorzugt um maximal 50 Gew.-%, weiter bevorzugt um maximal 20 Gew.-%, höher ist gegenüber dem initialen Feinanteil dieses Siebdurchmessers nach einer Siebzeit von 5 Minuten, t₅ₘᵢₙ; und/oder
- der Anteil an Partikeln mit einem Siebdurchmesser von 200 µm ≤ x < 800 µm nach einer Siebzeit von 60 Minuten, t₆₀ₘᵢₙ, um maximal 18 Gew.-%, bevorzugt um maximal 15 Gew.-%, weiter bevorzugt um maximal 12 Gew.-%, niedriger ist gegenüber dem initialen Anteil dieses Siebdurchmessers nach einer Siebzeit von 5 Minuten, t₅ₘᵢₙ, wobei die Vibrationssiebung auf einem Siebturm mit Siebböden von 20 cm Siebboden-Durchmesser und bei einer Vibrationsamplitude von 80/min durchgeführt wird, und hierbei eine zu testende Ausgangsmenge der Magnesium-haltigen Kerne von 100 g verwendet wird, sowie pro Siebboden 1,0 g einer Siebhilfe in Form von abriebfesten Silicagel-Kugeln mit einem Durchmesser von 3 mm und einer Schüttdichte von 750 kg/m³,
so dass die Retardpartikel im Industrie-Maßstab von ≥ 50 kg herstellbar sind.

2. Die Zusammensetzung nach einem der vorhergehenden Ansprüche, wobei die Magnesium-haltigen Kerne der Retardpartikel eine unimodale Partikelgrößenverteilung aufweisen; optional eine enge Partikelgrößenverteilung, bei welcher der Quotient (D90-D10) / D50 unter 1,60 liegt, bevorzugt unter 1,50, und weiter bevorzugt unter 1,40.

3. Die Zusammensetzung nach einem der vorhergehenden Ansprüche, wobei die Magnesiumverbindung in den Retardpartikeln ausgewählt ist aus Magnesiumoxid (MgO), Magnesiumcarbonat (MgCO₃), Magnesiumchlorid (MgCl₂), Magnesiumhydrogenphosphat (MgHPO₄) und/oder Magnesiumacetat (Mg(CH₃COO)₂).

4. Die Zusammensetzung nach einem der vorhergehenden Ansprüche, wobei die Retardpartikel der Komponente (a), und optional die nicht-retardierten Partikel der Komponente (b), eine mediane Teilchengröße (D50), gemessen mittels dynamischer Bildanalyse, von ≤ 500 µm aufweisen, bevorzugt ≤ 450 µm, weiter bevorzugt ≤ 400 µm.

5. Methode zur Charakterisierung eines Magnesium-haltigen Kerns für die Herstellung einer festen, lipid-überzogenen pharmazeutischen oder nutrazeutischen Zusammen-setzung in der Wirbelschicht, wobei der Kern eine Magnesiumverbindung enthält oder aus dieser besteht, und wobei die Methode **dadurch gekennzeichnet ist, dass** diese mittels einer Vibrationssiebung auf einem Siebturm mit Siebböden von 10-22 cm Siebboden-Durchmesser und bei einer Vibrationsamplitude von 50/min bis 100/min durchgeführt wird, und hierbei eine zu testende Ausgangsmenge der Magnesium-haltigen Kerne von 50-150 g verwendet wird, sowie pro Siebboden 0,5-2,0 g einer Siebhilfe in Form von abriebfesten Silicagel-Kugeln mit einem Durchmesser von 2-6 mm und einer Schüttdichte von 650-850 kg/m³, und
dadurch, dass Magnesium-haltige Kerne, welche für die Herstellung der festen, lipid-überzogenen pharmazeutischen oder nutrazeutischen Zusammensetzung in der Wirbelschicht als geeignet charakterisiert werden, in diesem Test eine solche Abriebfestigkeit aufweist, dass nach der Vibrationssiebung über einen Zeitraum von 60 Minuten:
- der Feinanteil an Partikeln mit einem Siebdurchmesser von < 200 µm nach einer Siebzeit von 60 Minuten, t₆₀ₘᵢₙ, um maximal 100 Gew.-%, bevorzugt um maximal 50 Gew.-%, weiter bevorzugt um maximal 20 Gew.-%, höher ist gegenüber dem initialen Feinanteil dieses Siebdurchmessers nach einer Siebzeit von 5 Minuten, t₅ₘᵢₙ; und/oder
- der Anteil an Partikeln mit einem Siebdurchmesser von 200 µm ≤ x < 800 µm nach einer Siebzeit von 60 Minuten, t₆₀ₘᵢₙ, um maximal 18 Gew.-%, bevorzugt um maximal 15 Gew.-%, weiter bevorzugt um maximal 12 Gew.-%, niedriger ist gegenüber dem initialen Anteil dieses Siebdurchmessers nach einer Siebzeit von 5 Minuten, t₅ₘᵢₙ.

6. Die Methode nach Anspruch 5, wobei die Vibrationssiebung bei einer Vibrationsamplitude von 80/min sowie mit einer zu testenden Ausgangsmenge der Magnesium-haltigen Kerne von 100 g durchgeführt wird.

7. Die Methode nach einem der vorhergehenden Ansprüche, wobei die Siebhilfe einen Durchmesser von 3 mm sowie eine Schüttdichte von 750 kg/m³ aufweist.

8. Die Methode nach einem der vorhergehenden Ansprüche, wobei pro Siebboden je 1 g der Siebhilfe verwendet wird.

9. Die Methode nach einem der vorhergehenden Ansprüche, wobei der Siebturm mit Sieben mit einem Siebdurchmesser von 800 µm, 600 µm, 500 µm, 400 µm, 300 µm, 200 µm, und 100 µm, sowie einer Bodenschale ausgestattet ist.

10. Die Methode nach einem der vorhergehenden Ansprüche, wobei die Magnesium-verbindung in den Magnesium-haltigen Kernen ausgewählt ist aus Magnesium-oxid (MgO), Magnesiumcarbonat (MgCO₃), Magnesiumchlorid (MgCl₂), Magnesiumhydrogenphosphat (MgHPO₄) und/oder Magnesiumacetat (Mg(CH₃COO)₂).

11. Verfahren zur Herstellung einer festen pharmazeutischen oder nutrazeutischen Zusammensetzung nach den Ansprüchen 1 bis 4 im Industrie-Maßstab von ≥ 50 kg, umfassend die folgenden Schritte:
(i) Bereitstellen eines Magnesium-haltigen Kerns, wobei der Kern eine Magnesium-verbindung enthält oder aus dieser besteht;
(ii) Bereitstellen eines geschmolzenen Überzugsmaterials enthaltend ein Lipid;
(iii) Fluidisieren der Magnesium-haltigen Kerne;
(iv) Besprühen der fluidisierten Magnesium-haltigen Kerne mit dem geschmolzenen Überzugsmaterial;
(v) Abkühlen der überzogenen Magnesium-haltigen Kerne, so dass das Lipid erstarrt und Retardpartikel gemäß Komponente (a) erhalten werden, welche das enthaltene Magnesium in retardierter Weise freisetzen; und
(vi) Mischen der so als Komponente (a) erhaltenen Retardpartikel mit den folgenden weiteren Komponenten:
(b) nicht-retardierte Partikel mit einem zweiten Magnesium-haltigen Kern, wobei der Partikel eine Magnesiumverbindung enthält oder aus dieser besteht, und wobei die Partikel das enthaltene Magnesium in nicht-retardierter Weise freisetzen;
(c) einen oder mehrere wasserlösliche Hilfsstoffe ausgewählt aus der Gruppe der Zucker, Zuckeralkohole und Oligosaccharide; und
(d) optional weiteren Hilfsstoffen;
**dadurch gekennzeichnet, dass** mindestens die Magnesium-haltigen Kerne der Retardpartikel der Komponente (a), bzw. eine Stichprobe hiervon, gemäß der Charakterisierungsmethode nach den Ansprüchen 5 bis 10 für die Herstellung der festen, lipidüberzogenen pharmazeutischen oder nutrazeutischen Zusammensetzung im Industrie-Maßstab von ≥ 50 kg geeignet sind, sprich eine solche Abriebfestigkeit aufweisen, dass nach einer Vibrationssiebung über einen Zeitraum von 60 Minuten:
der Feinanteil an Partikeln mit einem Siebdurchmesser von < 200 µm nach einer Siebzeit von 60 Minuten, t₆₀ₘᵢₙ, um maximal 100 Gew.-%, bevorzugt um maximal 50 Gew.-%, weiter bevorzugt um maximal 20 Gew.-%, höher ist gegenüber dem initialen Feinanteil dieses Siebdurchmessers nach einer Siebzeit von 5 Minuten, t₅ₘᵢₙ; und/oder
der Anteil an Partikeln mit einem Siebdurchmesser von 200 µm ≤ x < 800 µm nach einer Siebzeit von 60 Minuten, t₆₀ₘᵢₙ, um maximal 18 Gew.-%, bevorzugt um maximal 15 Gew.-%, weiter bevorzugt um maximal 12 Gew.-%, niedriger ist gegenüber dem initialen Anteil dieses Siebdurchmessers nach einer Siebzeit von 5 Minuten, t₅ₘᵢₙ.

12. Feste pharmazeutische oder nutrazeutische Zusammensetzung in Form eines oralen Direktgranulats mit mindestens dualer Wirkstofffreisetzung, hergestellt durch das Verfahren nach Anspruch 11.

## Claims

1. A solid pharmaceutical or nutraceutical composition in the form of an oral direct granulate with at least dual active ingredient release, containing the following components:
- coated retard particles with a magnesium-containing core and a lipid coating, wherein the core contains or consists of a magnesium compound, and wherein the retard particles release the contained magnesium in a retard manner;
- non-retard magnesium-containing particles, wherein the particles contain or consist of a magnesium compound, and wherein the particles release the magnesium contained therein in a non-retard manner;
- one or more water-soluble excipients selected from the group of sugars, sugar alcohols, and oligosaccharides; and
- optionally, one or more additional excipients;
**characterized in that** the retard particles are produced in a fluidized bed melt coating process, and **in that** at least the magnesium-containing cores of the retard particles of component (a), or a sample thereof, have such abrasion resistance that, after vibration screening over a period of 60 minutes:
- the fine fraction of particles with a sieve diameter of < 200 µm after a sieving time of 60 minutes, t₆₀ₘᵢₙ, is at most 100 wt.%, preferably at most 50 wt. %, more preferably at most 20 wt. %, higher compared to the initial fine fraction of this sieve diameter after a sieving time of 5 minutes, t₅ₘᵢₙ; and/or
- the fraction of particles with a sieve diameter of 200 µm ≤ x < 800 µm after a sieving time of 60 minutes, t₆₀ₘᵢₙ, is at most 18 wt.%, preferably at most 15 wt.%, more preferably at most 12 wt.%, lower compared to the initial fraction of this sieve diameter after a sieving time of 5 minutes, t₅ₘᵢₙ,
wherein the vibration sieving is carried out on a sieve tower with sieve bottoms having a diameter of 20 cm and at a vibration amplitude of 80/min, and wherein an initial quantity of the magnesium containing cores of 100 g is used, as well as 1.0 g per sieve bottom of a sieving aid in the form of abrasion-resistant silica gel balls with a diameter of 3 mm and a bulk density of 750 kg/m³,
so that the retard particles can be produced on an industrial scale of ≥ 50 kg.

2. The composition according to one of the preceding claims, wherein the magnesium-containing cores of the retard particles have a unimodal particle size distribution; optionally a narrow particle size distribution, wherein the quotient (D90-D10) / D50 is less than 1.60, preferably less than 1.50, and more preferably less than 1.40.

3. The composition according to one of the preceding claims, wherein the magnesium compound in the retard particles is selected from magnesium oxide (MgO), magnesium carbonate (MgCO₃), magnesium chloride (MgCl₂), magnesium hydrogen phosphate (MgHPO₄), and/or magnesium acetate (Mg(CH₃COO)₂).

4. The composition according to one of the preceding claims, wherein the retard particles of component (a), and optionally the non-retard particles of component (b), have a median particle size (D50), measured by dynamic image analysis, of ≤ 500 µm, preferably ≤ 450 µm, more preferably ≤ 400 µm.

5. A method for characterizing a magnesium-containing core for the production of a solid lipid-coated pharmaceutical or nutraceutical composition in a fluidized bed, wherein the core contains or consists of a magnesium compound, and wherein the method is **characterized in that** it is carried out by means of vibration sieving on a sieving tower with sieve bottoms having a diameter of 10 to 22 cm and at a vibration amplitude of 50/min to 100/min, and wherein a to be tested initial quantity of 50 to 150 g of the magnesium-containing cores is used, as well as 0.5 to 2.0 g per sieve bottom of a sieving aid in the form of abrasion-resistant silica gel balls with a diameter of 2 to 6 mm and a bulk density of 650 to 850 kg/m³, and
**in that** magnesium-containing cores, which are characterized as suitable for the production of the solid lipid-coated pharmaceutical or nutraceutical composition in the fluidized bed exhibit such abrasion resistance in this test that, after vibration sieving over a period of 60 minutes:
- the fine fraction of particles with a sieve diameter of < 200 µm after a sieving time of 60 minutes, t₆₀ₘᵢₙ, is at most 100 wt.%, preferably at most 50 wt.%, more preferably at most 20 wt.%, higher compared to the initial fine fraction of this sieve diameter after a sieving time of 5 minutes, t₅ₘᵢₙ; and/or
- the fraction of particles with a sieve diameter of 200 µm ≤ x < 800 µm after a sieving time of 60 minutes, t₆₀ₘᵢₙ, is at most 18 wt.%, preferably at most 15 wt.%, more preferably at most 12 wt.%, lower compared to the initial fraction of this sieve diameter after a sieving time of 5 minutes, t₅ₘᵢₙ.

6. The method according to claim 5, wherein the vibration sieving is carried out at a vibration amplitude of 80/min and with a to be tested initial quantity of magnesium-containing cores of 100 g.

7. The method according to one of the preceding claims, wherein the sieving aid has a diameter of 3 mm and a bulk density of 750 kg/m³.

8. The method according to one of the preceding claims, wherein 1 g of the sieving aid is used per sieve bottom.

9. The method according to one of the preceding claims, wherein the sieving tower is equipped with sieves having a sieve diameter of 800 µm, 600 µm, 500 µm, 400 µm, 300 µm, 200 µm, and 100 µm, as well as a bottom tray.

10. The method according to one of the preceding claims, wherein the magnesium compound in the magnesium-containing cores is selected from magnesium oxide (MgO), magnesium carbonate (MgCO₃), magnesium chloride (MgCl₂), magnesium hydrogen phosphate (MgHPO₄), and/or magnesium acetate (Mg(CH₃COO)₂).

11. A method for producing a solid pharmaceutical or nutraceutical composition according to claims 1 to 4 on an industrial scale of ≥ 50 kg, comprising the following steps:
(i) providing a magnesium-containing core, wherein the core contains or consists of a magnesium compound;
(ii) providing a molten coating material containing a lipid;
(iii) fluidizing the magnesium-containing cores;
(iv) spraying the fluidized magnesium-containing cores with the molten coating material;
(v) cooling the coated magnesium-containing cores so that the lipid solidifies and retard particles are obtained according to component (a), which release the contained magnesium in a retard manner; and
(vi) mixing the retard particles thus obtained as component (a) with the following further components:
(b) non-retard particles with a second magnesium-containing core, wherein the particle contains or consists of a magnesium compound, and wherein the particles release the contained magnesium in a non-retard manner;
(c) one or more water-soluble excipients selected from the group consisting of sugars, sugar alcohols, and oligosaccharides; and
(d) optionally, further excipients;
**characterized in that** at least the magnesium-containing cores of the retard particles of component (a), or a sample thereof, are suitable for the production of the solid, lipid-coated pharmaceutical or nutraceutical composition on an industrial scale of ≥ 50 kg according to the characterization method of claims 5 to 10, i.e., they have such abrasion resistance that after vibration sieving over a period of 60 minutes:
the fine fraction of particles with a sieve diameter of < 200 µm after a sieving time of 60 minutes, t₆₀ₘᵢₙ, is at most 100 wt.%, preferably at most 50 wt.%, more preferably at most 20 wt.%, higher compared to the initial fine fraction of this sieve diameter after a sieving time of 5 minutes, t₅ₘᵢₙ; and/or
the fraction of particles with a sieve diameter of 200 µm ≤ x < 800 µm after a sieving time of 60 minutes, t₆₀ₘᵢₙ, is at most 18 wt.%, preferably at most 15 wt.%, more preferably at most 12 wt.%, lower compared to the initial fraction of this sieve diameter after a sieving time of 5 minutes, t₅ₘᵢₙ.

12. A solid pharmaceutical or nutraceutical composition in the form of an oral direct granulate with at least dual active ingredient release, produced by the method according to claim 11.

## Revendications

1. Composition pharmaceutique ou nutraceutique solide sous forme de granulés oraux directe avec au moins une double libération de principe actif, contenant les composants suivants :
- des particules à libération retardée enrobées, avec un noyau contenant du magnésium et un enrobage lipidique, le noyau contenant un composé de magnésium ou étant constitué de celui-ci, et les particules à libération retardée libérant le magnésium contenu de manière retardée ;
- des particules non retardées contenant du magnésium, les particules contenant un composé de magnésium ou étant constituées de celui-ci, et les particules libérant le magnésium contenu de manière non retardée ;
- un ou plusieurs excipients hydrosolubles choisis dans le groupe des sucres, des alcools de sucre et des oligosaccharides ; et
- éventuellement un ou plusieurs autres excipients ;
**caractérisé en ce que** les particules à libération retardée sont produites dans un processus d'enrobage par fusion dans un lit fluidisé, et **en ce qu'**au moins les noyaux contenant du magnésium des particules à libération retardée du composant (a), ou un échantillon de celles-ci, présentent une résistance à l'abrasion telle qu'après un tamisage par vibration pendant une période de 60 minutes :
- la fraction fine de particules d'un diamètre de tamisage < 200 µm après un temps de tamisage de 60 minutes, t₆₀ₘᵢₙ, est supérieure de 100 % en poids au maximum, de préférence de 50 % en poids au maximum, de préférence encore de 20 % en poids au maximum, par rapport à la fraction fine initiale de ce diamètre de tamisage après un temps de tamisage de 5 minutes, t₅ₘᵢₙ ; et/ou
- la fraction de particules d'un diamètre de tamisage de 200 µm ≤ x < 800 µm après un temps de tamisage de 60 minutes, t₆₀ₘᵢₙ, est inférieure de 18 % en poids au maximum, de préférence de 15 % en poids au maximum, de préférence encore de 12 % en poids au maximum, par rapport à la fraction initiale de ce diamètre de tamisage après un temps de tamisage de 5 minutes, t₅ₘᵢₙ,
le tamisage par vibration étant effectué sur une tour de tamisage avec des fonds de tamis d'un diamètre de 20 cm et une amplitude de vibration de 80/min, et qu'une quantité initiale à tester de noyaux contenant du magnésium de 100 g est utilisée, ainsi que 1,0 g par fond de tamis d'un adjuvant de tamisage sous forme de billes de gel de silice résistantes à l'abrasion d'un diamètre de 3 mm et d'une densité apparente de 750 kg/m³, de sorte que les particules à libération retardée peuvent être produites à l'échelle industrielle à partir de ≥ 50 kg.

2. Composition selon l'une des revendications précédentes, dans laquelle les noyaux contenant du magnésium des particules à libération retardée présentent une distribution granulométrique unimodale ; éventuellement une distribution granulométrique étroite, dans laquelle le quotient (D90-D10) / D50 est inférieur à 1,60, de préférence inférieur à 1,50 et de préférence encore inférieur à 1,40.

3. Composition selon l'une des revendications précédentes, dans laquelle le composé de magnésium dans les particules à libération retardée est choisi parmi l'oxyde de magnésium (MgO), le carbonate de magnésium (MgCO₃), le chlorure de magnésium (MgCl₂), hydrogénophosphate de magnésium (MgHPO₄) et/ou acétate de magnésium (Mg(CH₃COO)₂).

4. Composition selon l'une des revendications précédentes, dans laquelle les particules à libération retardée du composant (a) et, en option, les particules à libération non retardées du composant (b) ont une taille médiane de particules (D50), mesurée par analyse d'image dynamique, ≤ 500 µm, de préférence ≤ 450 µm, de préférence encore ≤ 400 µm.

5. Procédé de caractérisation d'un noyau contenant du magnésium pour la fabrication d'une composition pharmaceutique ou nutraceutique solide enrobée de lipides dans un lit fluidisé, le noyau contenant un composé de magnésium ou étant constitué de celui-ci, et le procédé étant **caractérisé en ce qu'**il est mis en œuvre au moyen d'un tamisage par vibration sur une tour de tamisage avec des fonds de tamis d'un diamètre de 10 à 22 cm et à une amplitude de vibration de 50/min à 100/min, et qu'une quantité initiale à tester de 50 à 150 g de noyaux contenant du magnésium est utilisée, ainsi que 0,5 à 2,0 g par fond de tamis d'un adjuvant de tamisage sous forme de billes de gel de silice résistantes à l'abrasion d'un diamètre de 2 à 6 mm et d'une densité apparente de 650 à 850 kg/m³, et
**en ce que** les noyaux contenant du magnésium, qui sont caractérisés comme étant appropriés pour la fabrication de la composition pharmaceutique ou nutraceutique solide enrobée de lipides dans le lit fluidisé, présentent dans ce test une résistance à l'abrasion telle qu'après le tamisage par vibration pendant une période de 60 minutes :
- la fraction fine de particules d'un diamètre de tamisage < 200 µm après un temps de tamisage de 60 minutes, t₆₀ₘᵢₙ, est supérieure de 100 % en poids au maximum, de préférence de 50 % en poids au maximum %, de préférence encore au maximum de 20 % en poids, par rapport à la fraction fine initiale de ce diamètre de tamisage après un temps de tamisage de 5 minutes, t₅ₘᵢₙ ; et/ou
- la fraction de particules d'un diamètre de tamisage de 200 µm ≤ x < 800 µm après un temps de tamisage de 60 minutes, t₆₀ₘᵢₙ, est inférieure de 18 % en poids au maximum, de préférence de 15 % en poids au maximum %, de préférence encore de 12 % en poids au maximum, par rapport à la proportion initiale de ce diamètre de tamisage après un temps de tamisage de 5 minutes, t₅ₘᵢₙ.

6. Procédé selon la revendication 5, dans lequel le tamisage par vibration est effectué à une amplitude de vibration de 80/min et avec une quantité initiale à tester de 100 g de noyaux contenant du magnésium.

7. Procédé selon l'une des revendications précédentes, dans laquelle l'aide au tamisage présente un diamètre de 3 mm et une densité apparente de 750 kg/m³.

8. Procédé selon l'une des revendications précédentes, dans laquelle 1 g d'aide au tamisage est utilisé par fond de tamis.

9. Procédé selon l'une des revendications précédentes, dans lequel la tour de tamisage est équipée de tamis d'un diamètre de 800 µm, 600 µm, 500 µm, 400 µm, 300 µm, 200 µm et 100 µm, ainsi que d'un bac de fond.

10. Procédé selon l'une des revendications précédentes, dans lequel le composé de magnésium dans les noyaux contenant du magnésium est choisi parmi l'oxyde de magnésium (MgO), le carbonate de magnésium (MgCO₃), chlorure de magnésium (MgCl₂), hydrogénophosphate de magnésium (MgHPO₄) et/ou acétate de magnésium (Mg(CH₃COO)₂).

11. Procédé de fabrication d'une composition pharmaceutique ou nutraceutique solide selon les revendications 1 à 4 à une échelle industrielle ≥ 50 kg, comprenant les étapes suivantes :
(i) fourniture d'un noyau contenant du magnésium, le noyau contenant un composé de magnésium ou étant constitué de celui-ci ;
(ii) fourniture d'un matériau d'enrobage fondu contenant un lipide ;
(iii) fluidisation des noyaux contenant du magnésium ;
(iv) pulvérisation du matériau d'enrobage fondu sur les noyaux contenant du magnésium fluidisés ;
(v) refroidir les noyaux contenant du magnésium enrobés de manière à ce que le lipide se solidifie et à obtenir des particules à libération retardée selon le composant (a), qui libèrent le magnésium contenu de manière retardée; et
(vi) mélanger les particules à libération retardée ainsi obtenues comme composant (a) avec les autres composants suivants :
(b) des particules non retardées avec un deuxième noyau contenant du magnésium, la particule contenant un composé de magnésium ou étant constituées de celui-ci, et les particules libérant le magnésium contenu de manière non retardée ;
(c) un ou plusieurs excipients hydrosolubles choisis dans le groupe des sucres, des alcools de sucre et des oligosaccharides ; et
(d) éventuellement d'autres excipients ;
**caractérisé en ce qu'**au moins les noyaux contenant du magnésium des particules à libération retardées du composant (a), ou un échantillon de ceux-ci, sont appropriés à la méthode de caractérisation selon les revendications 5 à 10 pour la fabrication à l'échelle industrielle de ≥ 50 kg de la composition pharmaceutique ou nutraceutique solide enrobée de lipides, c'est-à-dire qu'ils présentent une résistance à l'abrasion telle qu'après un tamisage par vibration pendant une période de 60 minutes:
la fraction fine des particules d'un diamètre de tamisage < 200 µm après un temps de tamisage de 60 minutes, t₆₀ₘᵢₙ, est supérieure de 100 % en poids au maximum, de préférence de 50 % en poids au maximum, et de préférence encore de 20 % en poids au maximum, par rapport à la fraction fine initiale de ce diamètre de tamisage après un temps de tamisage de 5 minutes, t₅ₘᵢₙ ; et/ou
la fraction de particules d'un diamètre de tamisage de 200 µm ≤ x < 800 µm après un temps de tamisage de 60 minutes, t₆₀ₘᵢₙ, est inférieure de 18 % en poids au maximum, de préférence de 15 % en poids au maximum, de préférence encore de 12 % en poids au maximum, par rapport à la fraction initiale de ce diamètre de tamisage après un temps de tamisage de 5 minutes, t₅ₘᵢₙ.

12. Composition pharmaceutique ou nutraceutique solide sous forme de granulés oraux directe avec au moins une double libération de principe actif, fabriquée selon le procédé selon la revendication 11.
